# EUROPÄISCHE PATENTSCHRIFT

(11) **EP 0 963 982 B1**
(45) Veröffentlichungstag und Bekanntmachung des Hinweises auf die Patenterteilung: **13.03.2002**
(21) Anmeldenummer: 99101072.9
(22) Anmeldetag: 25.01.1999
(51) Int. Cl.: C07D 333/20, C07D 307/52, C07D 207/335, C07D 345/00, A61K 7/13

(54) **Neue Diaminobenzol-Derivate und diese Verbindungen enthaltende Färbemittel**
New diaminobenzene derivatives and these componends containing colouring agents
Derivés nouveaux du diamino-benzène et colorants contenants ces composés

(30) Priorität: 19.03.1998 DE 19812058
(43) Veröffentlichungstag der Anmeldung: 15.12.1999
(73) Patentinhaber: Wella Aktiengesellschaft, 64295 Darmstadt (DE)
(72) Erfinder: Braun, Hans-Jürgen, Dr., 3182 Überstorf (CH); Chassot, Laurent, Dr., 1724 Praroman (CH)

(56) Entgegenhaltungen:
- EP-A- 0 891 765
- US-A- 3 644 399
- US-A- 4 149 848
- US-A- 5 019 130
- US-A- 5 876 464
- PATENT ABSTRACTS OF JAPAN vol. 14, no. 202 (C-713), 25 April 1990 & JP 02 042014 A (KAO CORP) 13 Februar 1990,

## Beschreibung

Die Erfindung betrifft neue p-Diaminobenzol-Derivate sowie diese Verbindungen enthaltende Mittel zum Färben von Keratinfasem.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol und 1,4-Diaminobenzol eingesetzt, während als Kupplersubstanzen beispielsweise Resorcin, 4-Chlorresorcin, 1-Naphthol, 3-Aminophenol und Derivate des m-Phenylendiamins zu nennen sind.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwicklersubstanzen und Kupplersubstanzen eine breite Palette verschiedener Farbnuancen erzeugt werden kann.

Mit den derzeit eingesetzten Färbemitteln ist es jedoch nicht möglich, die vorgenannten Anforderungen in allen Punkten zu erfüllen.

Es besteht daher weiterhin ein Bedürfnis nach neuen Entwicklersubstanzen, welche die vorgenannten Anforderung in besonderem Masse erfüllen.

Hierzu wurde nun überraschenderweise gefunden, daß neue p-Diaminobenzol-Derivate gemäß der allgemeinen Formel (I) die an Entwicklersubstanzen gestellten Anforderungen in besonders hohem Masse erfüllen. So werden unter Verwendung dieser Entwicklersubstanzen mit den meisten bekannten Kupplersubstanzen farbstarke Farbnuancen erhalten, die außerordentlich lichtecht und waschecht sind.

Gegenstand der vorliegende Erfindung sind daher p-Diaminobenzol-Derivate der allgemeinen Formel (I) oder deren physiologisch verträgliche, wasserlösliche Salze, 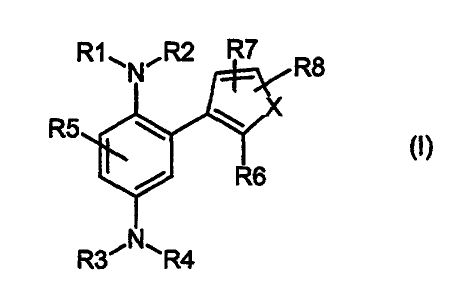 worin
**X** gleich Sauerstoff, Schwefel, Seien oder N-R9 ist;
**R1,R2,R3** und **R4** unabhängig voneinander Wasserstoff, eine C₁-C₆-Alkylgruppe, eine C₁-C₄-Hydroxyalkylgruppe, eine C₂-C₄ Dihydroxyalkylgruppe oder eine C₁-C₄-Alkoxy-(C₁-C₂)-alkylgruppe darstellen oder R1und R2 beziehungsweise R3 und R4 einen viergliedrigen bis achtgliedrigen aliphatischen Ring bilden, wobei mindestens 2 der Reste R1 bis R4 Wasserstoff darstellen;
**R5** gleich Wasserstoff, einem Halogenatom, einer C₁-C₄-Alkylgruppe, einer C₁-C₄-Hydroxyalkylgruppe oder einer C₁-C₄-Alkoxygruppe ist;
**R6** gleich Wasserstoff, einer Hydroxygruppe, einem Halogenatom, einer Cyanogruppe, einer C₁-C₄-Alkoxygruppe, einer C₁-C₆-Alkylgruppe, einer C₁-C₄-Alkylthioethergruppe, einer Mercaptogruppe, einer Nitrogruppe, einer Aminogruppe, einer Alkylaminogruppe, einer Dialkylaminogruppe, einer -C(O)H-Gruppe, einer -C(O)CH₃-Gruppe, einer -C(O)CF₃-Gruppe, einer -Si(CH₃)₃-Gruppe, einer C₁-C₄-Hydroxyalkylgruppe, einer C₃-C₄-Dihydroxyalkylgruppe, einer -CH=CHR10-Gruppe, einer -(CH₂)ₚ-CO₂R11-Gruppe oder einer -(CH₂)ₚ-R12-Gruppe mit p=1,2,3 oder 4, einer -C(R13)=NR14-Gruppe oder einer C(R16)H-NR17R18-Gruppe ist;
**R7** und **R8** unabhänging voneinander gleich Wasserstoff, einem Halogenatom, einer Cyanogruppe, einer Hydroxygruppe, einer C₁-C₄ -Alkoxygruppe, einer C₁-C₆ Alkylgruppe, einer C₁-C₄-Alkylthioethergruppe, einer Mercaptogruppe, einer Nitrogruppe, einer Aminogruppe, einer Alkylaminogruppe, einer Dialkylaminogruppe, einer -C(O)H-Gruppe, einer -C(O)CH₃-Gruppe, einer -C(O)CF₃-Gruppe, einer -Si(CH₃)₃-Gruppe, einer C₁-C₄-Hydroxyalkylgruppe, einer C₃-C₄-Dihydroxyalkylgruppe, einer -CH=CHR10-Gruppe, einer-(CH₂)ₚ-CO₂R11-Gruppe oder einer -(CH₂)ₚ-R12-Gruppe mit p=1,2,3 oder 4, einer-C(R13)=NR14-Gruppe oder einer C(R16)H-NR17R18-Gruppe sind;
**R9** gleich Wasserstoff, einer C₁-C₆-Alkylgruppe, einer C₁-C₄-Hydroxyalkylgruppe, einer Phenylgruppe oder einer Acetylgruppe ist;
**R10** gleich Wasserstoff, einer Hydroxygruppe, einer Nitrogruppe, einer Aminogruppe, einer -CO₂R11-Gruppe oder einer -C(O)CH₃-Gruppe ist;
**R11, R13 und R16** unabhänging voneinander gleich Wasserstoff oder einer C₁-C₄-Alkylgruppe sind;
**R12** gleich einer Aminogruppe oder einer Nitrilgruppe ist;
**R14, R17 und R18** unabhängig voneinander gleich Wasserstoff, einer Hydroxygruppe, einer C₁-C₄-Alkylgruppe, einer C₁-C₄-Hydroxyalkylgruppe, einer C₃-C₄-Dihydroxyalkylgruppe oder einem Rest der Formel (II) sind 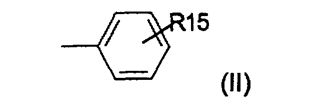 und
**R15** gleich Wasserstoff, einer Aminogruppe oder einer Hydroxygruppe ist.

Als Verbindungen der Formel (I) können beispielweise die folgenden Verbindungen genannt werden:
2,5-Diamino-(3-thienyl)benzol; 2-Amino-5-methylamino-(3-thienyl)benzol; 5-Amino-2-methylamino-(3-thienyl)benzol; 2-Amino-5-dimethylamino-(3-thienyl)benzol; 5-Ammo-2-dimethylammo-(3-thienyl)benzol; 2-Amino-5-(2-hydroxyethyl)amino-(3-thienyl)benzol; 5-Amino-2-(2-hydroxyethyl)amino-(3-thienyl)benzol; 2-Amino-5-di(2-hydroxyethyl)amino-(3-thienyl)benzol; 5-Amino-2-di(2-hydroxyethyl)amino-(3-thienyl)benzol; 2-Amino-5-(2-hydroxyethyl)methylamino-(3-thienyl)benzol; 5-Amino-2-(2-hydroxyethyl)-methylamino-(3-thienyl)benzol; 2-Amino-5-(2,3-dihydroxypropyl)amino-(3-thienyl)benzol; 5-Amino-2-(2,3-dihydroxypropyl)amino-(3-thienyl)benzol; 2-Amino-5-di(2,3-dihydroxypropyl)amino-(3-thienyl)benzol; 5-Amino-2-di(2,3-dihydroxypropyl)amino-(3-thienyl)benzol; 2-Amino-5-(2,3-dihydroxypropyl)methylamino-(3-thienyl)benzol; 5-Amino-2-(2,3-dihydroxypropyl)-methylamino-(3-thienyl)benzol; 2-Amino-5-(2-methoxyethyl)amino-(3-thienyl)benzol; 5-Amino-2-(2-methoxyethyl)amino-(3-thienyl)benzol; 2-Amino-5-di(2-methoxyethyl)amino-(3-thienyl)benzol; 5-Amino-2-di(2-methoxyethyl)amino-(3-thienyl)benzol; 2-Amino-5-(2-methoxyethyl)-methylamino-(3-thienyl)benzol; 5-Amino-2-(2-methoxyethyl)methylamino-(3-thienyl)benzol; 2-Amino-5-methylamino-(3-furyl)benzol; 5-Amino-2-methylamino-(3-furyl)benzol; 2-Amino-5-dimethylamino-(3-furyl)benzol; 5-Amino-2-dimethylamino-(3-furyl)benzol; 2-Amino-5-(2-hydroxyethyl)amino-(3-furyl)benzol; 5-Amino-2-(2-hydroxyethyl)amino-(3-furyl)benzol; 2-Amino-5-di(2-hydroxyethyl)amino-(3-furyl)benzol; 5-Amino-2-di(2-hydroxyethyl)amino-(3-furyl)benzol; 2-Amino-5-(2-hydroxyethyl)methylamino-(3-furyl)benzol; 5-Amino-2-(2-hydroxyethyl)-methylamino-(3-furyl)benzol; 2-Amino-5-(2,3-dihydroxypropyl)amino-(3-furyl)benzol; 5-Amino-2-(2,3-dihydroxypropyl)amino-(3-furyl)benzol; 2-Amino-5-di(2,3-dihydroxypropyl)amino-(3-furyl)benzol; 5-Amino-2-di(2,3-dihydroxypropyl)amino-(3-furyl)benzol; 2-Amino-5-(2,3-dihydroxypropyl)methylamino-(3-furyl)benzol; 5-Amino-2-(2,3-dihydroxypropyl)-methylamino-(3-furyl)benzol; 2-Amino-5-(2-methoxyethyl)amino-(3-furyl)benzol; 5-Amino-2-(2-methoxyethyl)amino-(3-furyl)benzol; 2-Amino-5-di(2-methoxyethyl)amino-(3-furyl)benzol; 5-Amino-2-di(2-methoxyethyl)amino-(3-furyl)benzol; 2-Amino-5-(2-methoxyethyl)-methylamino-(3-furyl)benzol; 5-Amino-2-(2-methoxyethyl)methylamino-(3-furyl)benzol; 2-Amino-5-methylamino-(pyrrol-3-yl)benzol; 5-Amino-2-methylamino-(pyrrol-3-yl)benzol; 2-Amino-5-dimethylamino-(pyrrol-3-yl)benzol; 5-Amino-2-dimethylamino-(pyrrol-3-yl)benzol; 2-Amino-5-(2-hydroxyethyl)amino-(pyrol-3-yl)benzol; 5-Amino-2-(2-hydroxyethyl)amino-(pyrrol-3-yl)benzol; 2-Amino-5-di(2-hydroxyethyl-)amino-(pyrrol-3-yl)benzol; 5-Amino-2-di(2-hydroxyethyl)amino-(pyrrol-3-yl)benzol; 2-Amino-5-(2-hydroxyethyl)methylamino-(pyrrol-3-yl)benzol; 5-Amino-2-(2-hydroxyethyl)methylamino-(pyrrol-3-yl)benzol; 2-Amino-5-(2,3-dihydroxypropyl)amino-(pyrrol-3-yl)benzol; 5-Amino-2-(2,3-dihydroxypropyl)amino-(pyrrol-3-yl)benzol; 2-Amino-5-di(2,3-dihydroxypropyl)amino-(pyrrol-3-yl)benzol; 5-Amino-2-di(2,3-dihydroxypropyl)amino-(pyrrol-3-yl)benzol; 2-Amino-5-(2,3-dihydroxypropyl)methylamino-(pyrrol-3-yl)benzol; 5-Amino-2-(2,3-dihydroxypropyl)methylamino-(pyrrol-3-yl)benzol; 2-Amino-5-(2-methoxyethyl)amino-(pyrrol-3-yl)benzol; 5-Amino-2-(2-methoxyethyl)amino-(pyrrol-3-yl)benzol; 2-Amino-5-di(2-methoxyethyl)amino-(pyrrol-3-yl)benzol; 5-Amino-2-di(2-methoxyethyl)-amino-(pyrrol-3-yl)benzol; 2-Amino-5-(2-methoxyethyl)methylamino-(pyrrol-3-yl)benzol; 5-Amino-2-(2-methoxyethyl)methylamino-(pyrrol-3-yl)benzol; 2-Amino-5-methylamino-(1-methyl-1H-pyrrol-3-yl)benzol; 5-Amino-2-methylamino-(1-methyl-1H-pyrrol-3-yl)benzol; 2-Amino-5-dimethylamino-(1-methyl-1H-pyrrol-3-yl)benzol; 5-Amino-2-dimethylamino-(1-methyl-1H-pyrrol-3-yl)benzol; 2-Amino-5-(2-hydroxyethyl)amino-(1-methyl-1H-pyrrol-3-yl)benzol; 5-Amino-2-(2-hydroxyethyl)amino-(1-methyl-1H-pyrrol-3-yl)benzol; 2-Amino-5-di(2-hydroxyethyl)amino-(1-methyl-1H-pyrrol-3-yl)benzol; 5-Amino-2-di(2-hydroxyethyl)amino-(1-methyl-1H-pyrrol-3-yl)benzol; 2-Amino-5-(2-hydroxyethyl)methylamino-(1-methyl-1H-pyrrol-3-yl)benzol; 5-Amino-2-(2-hydroxyethyl)methylamino-(1-methyl-1H-pynol-3-yl)benzol; 2-Amino-5-(2,3-dihydroxypropyl)amino-(1-methyl-1H-pyrrol-3-yl)benzol; 5-Amino-2-(2,3-dihydroxypropyl)amino-(1-methyl-1H-pyrrol-3-yl)benzol; 2-Amino-5-di(2,3-dihydroxypropyl)amino-(1-methyl-1H-pyrrol-3-yl)benzol; 5-Amino-2-di(2,3-dihydroxypropyl)amino-(1-methyl-1H-pyrrol-3-yl)benzol; 2-Amino-5-(2,3-dihydroxypropyl)methylamino-(1-methyl-1H-pyrrol-3-yl)benzol; 5-Amino-2-(2,3-dihydroxypropyl)-methylamino-(1-methyl-1H-pyrrol-3-yl)benzol; 2-Amino-5-(2-methoxyethyl)amino-(1-methyl-1H-pyrrol-3-yl)benzol; 5-Amino-2-(2-methoxyethyl)amino-(1-methyl-1H-pyrrol-3-yl)benzol; 2-Amino-5-di(2-methoxyethyl)amino-(1-methyl-1H-pyrrol-3-yl)benzol; 5-Amino-2-di(2-methoxyethyl)amino-(1-methyl-1H-pyrrol-3-yl)benzol; 2-Amino-5-(2-methoxyethyl)methylamino-(1-methyl-1H-pyrrol-3-yl)benzol; 5-Amino-2-(2-methoxyethyl)methylamino-(1-methyl-1 H-pyrrol-3-yl)benzol; 2,5-Diamino-3-methyl-(3-thienyl)benzol; 2-Amino-5-di(2-hydroxyethyl)amino-3-methyl-(3-thienyl)benzol; 5-Amino-2-di(2-hydroxyethyl)amino-3-methyl-(3-thienyl)-benzol; 2,5-Diamino-3-chlor-(3-thienyl)benzol; 2-Amino-5-di(2-hydroxyethyl)amino-3-chlor-(3-thienyl)benzol; 5-Amino-2-di(2-hydroxyethyl)amino-3-chlor-(3-thienyl)benzol; 2,5-Diamino-4-methyl-(3-thienyl)benzol; 2-Amino-5-di(2-hydroxyethyl)amino-4-methyl-(3-thienyl)benzol; 5-Amino-2-di(2-hydroxyethyl)amino-4-methyl-(3-thienyl)benzol; 2,5-Diamino-4-chlor-(3-thienyl)benzol; 2-Amino-5-di(2-hydroxyethyl)amino-4-chlor-(3-thienyl)-benzol; 5-Amino-2-di(2-hydroxyethyl)amino-4-chlor-(3-thienyl)benzol; 2,5-Diamino-5-methyl-(3-thienyl)benzol;2-Amino-5-di(2-hydroxyethyl)-amino-5-methyl-(3-thienyl)benzol; 5-Amino-2-di(2-hydroxyethyl)amino-5-methyl-(3-thienyl)benzol; 2,5-Diamino-5-chlor-(3-thienyl)benzol; 2-Amino-5-di(2-hydroxyethyl)amino-5-chlor-(3-thienyl)benzol; 5-Amino-2-di(2-hydroxyethyl)amino-5-chlor-(3-thienyl)benzol; 2,5-Diamino-3-methyl-(3-furyl)benzol; 2-Amino-5-di(2-hydroxyethyl)amino-3-methyl-(3-furyl)-benzol; 5-Amino-2-di(2-hydroxyethyl)amino-3-methyt-(3-furyl)benzol; 2,5-Diamino-3-chlor-(3-furyl)benzol; 2-Amino-5-di(2-hydroxyethyl)-amino-3-chlor-(3-furyl)benzol; 5-Amino-2-di(2-hydroxyethyl)amino-3-chlor-(3-furyl)benzol; 2,5-Diamino-4-methyl-(3-furyl)benzol; 2-Amino-5-di(2-hydroxyethyl)amino-4-methyl-(3-furyl)benzol; 5-Amino-2-di(2-hydroxyethyl)amino-4-methyl-(3-furyl)benzol; 2,5-Diamino-4-chlor-(3-furyl)benzol; 2-Amino-5-di(2-hydroxyethyl)amino-4-chlor-(3-furyl)-benzol; 5-Amino-2-di(2-hydroxyethyl)amino-4-chlor-(3-furyl)benzol; 2,5-Diamino-5-methyl-(3-furyl)benzol; 2-Amino-5-di(2-hydroxyethyl)-amino-5-methyl-(3-furyl)benzol; 5-Amino-2-di(2-hydroxyethyl)amino-5-methyl-(3-furyl)benzol; 2,5-Diamino-5-chlor-(3-furyl)benzol; 2-Amino-5-di(2-hydroxyethyl)amino-5-chlor-(3-furyl)benzol; 5-Amino-2-di(2-hydroxyethyl)amino-5-chlor-(3-furyl)benzol; 2,5-Diamino-3-methyl-(pyrrol-3-yl)benzol; 2-Amino-5-di(2-hydroxyethyl)amino-3-methyl-(pyrrol-3-yl)benzol; 5-Amino-2-di(2-hydroxyethyl)amino-3-methyl-(pyrrol-3-yl)benzol; 2,5-Diamino-3-Chlor-(pyrrol-3-yl)benzol; 2-Amino-5-di(2-hydroxyethyl)-amino-3-chlor-(pyrrol-3-yl)benzol; 5-Amino-2-di(2-hydroxyethyl)amino-3-chlor-(pyrrol-3-yl)benzol; 2,5-Diamino-4-methyl-(pyrrol-3-yl)benzol; 2-Amino-5-di(2-hydroxyethyl)amino-4-methyl-(pyrrol-3-yl)benzol; 5-Amino-2-di(2-hydroxyethyl)amino-4-methyl-(pyrrol-3-yl)benzol; 2,5-Diamino-4-chlor-(pyrrol-3-yl)benzol; 2-Amino-5-di(2-hydroxyethyl)-amino-4-chlor-(pyrrol-3-yl)benzol; 5-Amino-2-di(2-hydroxyethyl)amino-4-chlor-(pyrrol-3-yl)benzol; 2,5-Diamino-5-methyl-(pyrrol-3-yl)benzol; 2-Amino-5-di(2-hydroxyethyl)amino-5-methyl-(pyrrol-3-yl)benzol; 5-Amino-2-di(2-hydroxyethyl)amino-5-methyl-(pyrrol-3-yl)benzol; 2,5-Diamino-5-chlor-(pyrrol-3-yl)benzol; 2-Amino-5-di(2-hydroxyethyl)amino-5-chlor-(pyrrol-3-yl)benzol; 5-Amino-2-di(2-hydroxyethyl)amino-5-chlor-(pyrrol-3-yl)benzol; 2,5-Diamino-3-methyl-(1-methyl-1H-pyrrol-3-yl)benzol; 2-Amino-5-di(2-hydroxyethyl)amino-3-methyl-(1-methyl-1H-pyrrol-3-yl)benzol; 5-Amino-2-di(2-hydroxyethyl)amino-3-methyl-(1-methyl-1H-pyrrol-3-yl)-benzol; 2,5-Diamino-3-chlor-(1-methyl-1H-pyrrol-3-yl)benzol; 2-Amino-5-di(2-hydroxyethyl)amino-3-chlor-(1-methyl-1H-pyrrol-3-yl)benzol; 5-Amino-2-di(2-hydroxyethyl)amino-3-chlor-(1-methyl-1H-pyrrol-3-yl)benzol; 2,5-Diamino-4-methyl-(1-methyl-1H-pyrrol-3-yl)benzol; 2-Amino-5-di(2-hydroxyethyl)amino-4-methyl-(1-methyl-1H-pyrrol-3-yl)benzol; 5-Amino-2-di(2-hydroxyethyl)amino-4-methyl-(1-methyl-1H-pyrrol-3-yl)benzol; 2,5-Diamino-4-chlor-(1-methyl-1H-pyrrol-3-yl)benzol; 2-Amino-5-di(2-hydroxyethyl)amino-4-chlor-(1-methyl-1H-pyrrol-3-yl)benzol; 5-Amino-2-di(2-hydroxyethyl)amino-4-chloro-(1-methyl-1H-pyrrol-3-yl)benzol; 2,5-Diamino-5-methyl-(1-methyl-1H-pyrrol-3-yl)benzol; 2-Amino-5-di(2-hydroxyethyl)amino-5-methyl-(1-methyl-1H-pyrrol-3-yl)benzol; 5-Amino-2-di(2-hydroxyethyl)amino-5-methyl-(1-methyl-1H-pyrrol-3-yl)benzol; 2,5-Diamino-5-chlor-(1-methyl-1H-pyrrol-3-yl)benzol; 2-Amino-5-di(2-hydroxyethyl)amino-5-chlor-(1-methyl-1H-pyrrol-3-yl)benzol; 5-Amino-2-di(2-hydroxyethyl)amino-5-chlor-(1-methyl-1H-pyrrol-3-yl)benzol; 2,5-Diamino-(2-methyl-3-thienyl) benzol; 2-Amino-5-di(2-hydroxyethyl)-amino-(2-methyl-3-thienyl) benzol; 5-Amino-2-di(2-hydroxyethyl)amino-(2-methyl-3-thienyl) benzol; 2,5-Diamino-(2-methyl-3-furyl) benzol; 2-Amino-5-di(2-hydroxyethyl)amino-(2-methyl-3-furyl) benzol; 5-Amino-2-di(2-hydroxyethyl)amino-(2-methyl-3-furyl) benzol; 2,5-Diamino-(4-methyl-3-thienyl) benzol; 2-Amino-5-di(2-hydroxyethyl)amino-(4-methyl-3-thienyl) benzol; 5-Amino-2-di(2-hydroxyethyl)amino-(4-methyl-3-thienyl)- benzol; 2,5-Diamino-(4-methyl-3-furyl) benzol; 2-Amino-5-di(2-hydroxyethyl)amino-(4-methyl-3-furyl)benzol; 5-Amino-2-di(2-hydroxyethyl)-amino-(4-methyl-3-furyl) benzol; 2,5-Diamino-(5-methyl-3-thienyl)- benzol; 2-Amino-5-di(2-hydroxyethyl)amino-(5-methyl-3-thienyl)benzol; 5-Amino-2-di(2-hydroxyethyl)amino-(5-methyl-3-thienyl) benzol; 2,5-Diamino-(5-methyl-3-furyl)benzol; 2-Amino-5-di(2-hydroxyethyl)-amino-(5-methyl-3-furyl)benzol; 5-Amino-2-di(2-hydroxyethyl)amino-(5-methyl-3-furyl)benzol; 2,5-Diamino-(2-ethyl-3-thienyl)benzol; 2-Amino-5-di(2-hydroxyethyl)amino-(2-ethyl-3-thienyl)benzol; 5-Amino-2-di(2-hydroxyethyl)amino-(2-ethyl-3-thienyl)benzol; 2,5-Diamino-(2-ethyl-3-furyl)benzol; 2-Amino-5-di(2-hydroxyethyl)amino-(2-ethyl-3-furyl)- benzol; 5-Amino-2-di(2-hydroxyethyl)amino-(2-ethyl-3-furyl)benzol; 2,5-Diamino-(4-ethyl-3-thienyl)benzol; 2-Amino-5-di(2-hydroxyethyl)amino-(4-ethyl-3-thienyl)benzol; 5-Amino-2-di(2-hydroxyethyl)amino-(4-ethyl-3-thienyl)benzol; 2,5-Diamino-(4-ethyl-3-furyl)benzol; 2-Amino-5-di(2-hydroxyethyl)amino-(4-ethyl-3-furyl)benzol; 5-Amino-2-di(2-hydroxyethyl)amino-(4-ethyl-3-furyl)benzol; 2,5-Diamino-(5-ethyl-3-thienyl)benzol; 2-Amino-5-di(2-hydroxyethyl)amino-(5-ethyl-3-thienyl)benzol; 5-Amino-2-di(2-hydroxyethyl)amino-(5-ethyl-3-thienyl)benzol; 2,5-Diamino-(5-ethyl-3-furyl)benzol; 2-Amino-5-di(2-hydroxyethyl)amino-(5-ethyl-3-furyl)benzol; 5-Amino-2-di(2-hydroxyethyl)amino-(5-ethyl-3-furyl)benzol; 2,5-Diamino-(2-dimethylamino-3-thienyl)benzol; 2-Amino-5-di(2-hydroxyethyl)amino-(2-dimethylamino-3-thienyl)benzol; 5-Amino-2-di(2-hydroxyethyl)amino-(2-dimethylamino-3-thienyl)benzol; 2,5-Diamino-(2-dimethylamino-3-furyl)benzol; 2-Amino-5-di(2-hydroxyethyl)amino-(2-dimethylamino-3-fulyl)benzol; 5-Amino-2-di(2-hydroxyethyl)amino-(2-dimethylamino-3-furyl)benzol; 2,5-Diamino-(4-dimethylamino-3-thienyl)benzol; 2-Amino-5-di(2-hydroxyethyl)amino-(4-dimethylamino-3-thienyl)benzol; 5-Amino-2-di(2-hydroxyethyl)amino-(4-dimethylamino-3-thienyl)benzol; 2,5-Diamino-(4-dimethylamino-3-furyl)benzol; 2-Amino-5-di(2-hydroxyethyl)amino-(4-dimethylamino-3-furyl)benzol; 5-Amino-2-di(2-hydroxyethyl)amino-(4-dimethylamino-3-furyl)benzol; 2,5-Diamino-(5-dimethylamino-3-thienyl)benzol; 2-Amino-5-di(2-hydroxyethyl)amino-(5-dimethylamino-3-thienyl)benzol; 5-Amino-2-di(2-hydroxyethyl)amino-(5-dimethylamino-3-thienyl)benzol; 2,5-Diamino-(5-dimethylamino-3-furyl)benzol; 2-Amino-5-di(2-hydroxyethyl)amino-(5-dimethylamino-3-furyl)benzol; 5-Amino-2-di(2-hydroxyethyl)amino-(5-dimethylamino-3-furyl)benzol; 2,5-Diamino-(2-formyl-3-thienyl)benzol; 2-Amino-5-di(2-hydroxyethyl)amino-(2-formyl-3-thienyl)-benzol; 5-Amino-2-di(2-hydroxyethyl)amino-(2-formyl-3-thienyl)benzol; 2,5-Diamino-(2-formyl-3-furyl)benzol; 2-Amino-5-di(2-hydroxyethyl)-amino-(2-formyl-3-furyl)benzol; 5-Amino-2-di(2-hydroxyethyl)amino-(2-formyl-3-furyl)benzol; 2,5-Diamino-(4-formyl-3-thienyl)benzol; 2-Amino-5-di(2-hydroxyethyl)amino-(4-formyl-3-thienyl)benzol; 5-Amino-2-di(2-hydroxyethyl)amino-(4-formyl-3-thienyl)benzol; 2,5-Diamino-(4-formyl-3-furyl)benzol; 2-Amino-5-di(2-hydroxy-ethyl)amino-(4-formyl-3-furyl)benzol; 5-Amino-2-di(2-hydroxy-ethyl)amino-(4-formyl-3-furyl)benzol; 2,5-Diamino-(5-formyl-3-thienyl)benzol; 2-Amino-5-di(2-hydroxyethyl)amino-(5-formyl-3-thienyl)benzol; 5-Amino-2-di(2-hydroxyethyl)amino-(5-formyl-3-thienyl)benzol; 2,5-Diamino-(5-formyl-3-furyl)benzol; 2-Amino-5-di(2-hydroxyethyl)amino-(5-formyl-3-furyl)benzol; 5-Amino-2-di(2-hydroxy-ethyl)amino-(5-formyl-3-furyl)benzol; 2,5-Diamino-(2-acetyl-3-thienyl)benzol; 2-Amino-5-di(2-hydroxyethyl)amino-(2-acetyl-3-thienyl)- benzol; 5-Amino-2-di(2-hydroxyethyl)amino-(2-acetyl-3-thienyl)benzol; 2,5-Diamino-(2-acetyl-3-furyl)benzol; 2-Amino-5-di(2-hydroxyethyl)-amino-(2-acetyl-3-furyl)benzol; 5-Amino-2-di(2-hydroxyethyl)amino-(2-acetyl-3-furyl)benzol; 2,5-Diamino-(4-acetyl-3-thienyl)benzol; 2-Amino-5-di(2-hydroxyethyl)amino-(4-acetyl-3-thienyl)benzol; 5-Amino-2-di(2-hydroxyethyl)amino-(4-acetyl-3-thienyl)benzol; 2,5-Diamino-(4-acetyl-3-furyl)benzol; 2-Amino-5-di(2-hydroxyethyl)amino-(4-acetyl-3-furyl)benzol; 5-Amino-2-di(2-hydroxyethyl)amino-(4-acetyl-3-furyl)benzol; 2,5-Diamino-(5-acetyl-3-thienyl)benzol; 2-Amino-5-di(2-hydroxyethyl)amino-(5-acetyl-3-thienyl)benzol; 5-Amino-2-di(2-hydroxyethyl)amino-(5-acetyl-3-thienyl)benzol; 2,5-Diamino-(5-acetyl-3-furyl)benzol; 2-Amino-5-di(2-hydroxyethyl)amino-(5-acetyl-3-furyl)benzol; 5-Amino-2-di(2-hydroxyethyl)amino-(5-acetyl-3-furyl)benzol; 2,5-Diamino-(2-aminomethyl-3-thienyl)benzol; 2-Amino-5-di(2-hydroxyethyl)amino-(2-aminomethyl-3-thienyl)benzol; 5-Amino-2-di(2-hydroxyethyl)amino-(2-aminomethyl-3-thienyl)benzol; 2,5-Diamino-(2-aminomethyl-3-furyl)benzol; 2-Amino-5-di(2-hydraxyethyl)amino-(2-aminomethyl-3-furyl)benzol; 5-Amino-2-di(2-hydroxyethyl)amino-(2-aminomethyl-3-furyl)benzol; 2,5-Diamino-(4-aminomethyl-3-thienyl)-benzol; 2-Amino-5-di(2-hydroxyethyl)amino-(4-aminomethyl-3-thienyl)-benzol; 5-Amino-2-di(2-hydroxyethyl)amino-(4-aminomethyl-3-thienyl)-benzol; 2,5-Diamino-(4-aminomethyl-3-furyl)benzol; 2-Amino-5-di(2-hydroxyethyl)amino-(4-aminomethyl-3-furyl)benzol; 5-Amino-2-di(2-hydroxyethyl)amino-(4-aminomethyl-3-furyl)benzol; 2,5-Diamino-(5-aminomethyl-3-thienyl)benzol; 2-Amino-5-di(2-hydroxyethyl)amino-(5-aminomethyl-3-thienyl)benzol; 5-Amino-2-di(2-hydroxyethyl)amino-(5-aminomethyl-3-thienyl)benzol; 2,5-Diamino-(5-aminomethyl-3-furyl)-benzol; 2-Amino-5-di(2-hydroxyethyl)amino-(5-aminomethyl-3-furyl)-benzol; 5-Amino-2-di(2-hydroxyethyl)amino-(5-aminomethyl-3-furyl)-benzol; 2,5-Diamino-(2-(2-hydroxyethyl(imino))methylen-3-thienyl)benzol; 2-Amino-5-di(2-hydroxyethyl)amino-(2-(2-hydroxyethyl(imino))methylen-3-thienyl)benzol; 5-Amino-2-di(2-hydroxyethyl)amino-(2-(2-hydroxyethyl-(imino))-methylen-3-thienyl)benzol; 2,5-Diamino-(2-(2-hydroxyethyl-(imino))methylen-3-furyl)benzol; 2-Amino-5-di(2-hydroxy-ethyl)amino-(2-(2-hydroxyethyl(imino))methylen-3-furyl)benzol; 5-Amino-2-di(2-hydroxyethyl)amino-(2(2-hydroxyethyl(imino))methylen-3-furyl)benzol; 2,5-Diamino-(4-(2-hydroxyethyl(imino))methylen-3-thienyl)- benzol; 2-Amino-5-di(2-hydroxyethyl)amino-(4-(2-hydroxyethyl(imino))-methylen-3-thienyl)benzol; 5-Amino-2-di(2-hydroxyethyl)amino-(4-(2-hydroxyethyl-(imino))methylen-3-thienyl)benzol; 2,5-Diamino-(4-(2-hydroxyethyl-(imino))methylen-3-furyl)benzol; 2-Amino-5-di(2-hydroxy-ethyl)amino-(4-(2-hydroxyethyl(imino))methylen-3-furyl)benzol; 5-Amino-2-di(2-hydroxyethyl)amino-(4-(2-hydroxyethyl(imino))methylen-3-furyl)benzol; 2,5-Diamino-(5-(2-hydroxyethyl(imino))methylen-3-thienyl)-benzol; 2-Amino-5-di(2-hydroxyethyl)amino-(5-(2-hydroxyethyl(imino))-methylen-3-thienyl)benzol; 5-Amino-2-di(2-hydroxyethyl)amino-(5-(2-hydroxyethyl-(imino))methylen-3-thienyl)benzol; 2,5-Diamino-(5-(2-hydroxyethyl-(imino))methylen-3-furyl)benzol; 2-Amino-5-di(2-hydroxy-ethyl)amino-(5-(2-hydroxyethyl(imino))methylen-3-furyl)benzol und 5-Amino-2-di(2-hydroxyethyl)amino-(5-(2-hydroxyethyl(imino))methylen-3-furyl)benzol.

Bevorzugt sind Verbindungen der Formel (I), bei denen (i) eine oder mehrere der Restgruppen R5, R6, R7 und R8 gleich Wasserstoff sind und/oder (ii) R1, R2, R3 und R4 gleichzeitig Wasserstoff bedeuten und/oder (iii) R7 gleich Wasserstoff und R6 gleich Wasserstoff, -C(O)H,-C(O)CH₃, C₁-C₄-Alkyl oder C₁-C₄-Hydroxyalkyl (insbesondere R6=R7=Wasserstoff) ist.

Insbesondere sind die folgenden Verbindungen zu nennen:
2,5-Diamino-1-(5-chlor-3-thienyl)benzol; 2,5-Diamino-1-(3-furyl)benzol; 2,5-Diamino-1-(2-acetyl-3-thienyl)benzol; 2,5-Diamino-1-(pyrrol-3-yl)benzol; 2,5-Diamino-1-(2-formyl-3-thienyl)benzol; 2,5-Diamino-1-(4-formyl-3-thienyl)-benzol; 2,5-Diamino-1-(2-formyl-3-furyl)benzol; 2,5-Diamino-1-(4-formyl-3-furyl)benzol; 2,5-Diamino-1-(5-methyl-3-thienyl)benzol, 2,5-Diamino-1-(5-methyl-3-thienyl)benzol, 2,5-Diamino-1-(4-(4-nitropropenyl)-3-thienyl)benzol, 2,5-Diamino-1-(3-furyl)benzol, 2,5-Diamino-1-(4-formyl-3-thienyl)benzol, 2,5-Diamino-1-(4-(2-hydroxyethyl)aminomethyl-3-thienyl)-benzol, 2,5-Diamino-1-(4-pyrrolidinomethyl-3-thienyl)-benzol, 2,5-Diamino-1-(4-(dimethylaminoethyl)aminomethyl-3-thienyl)-benzol, 2,5-Diamino-1-(4-(2-methoxy-5-chlor-phenyl)aminomethyl-3-thlenyl)-benzol, 2,5-Diamino-1-(4-(3-ethoxypropyl)aminomethyl-3-thienyl)-benzol, 2,5-Diamino-1-(4-(methoxyethyl)aminomethyl-3-thienyl)-benzol, 2,5-Diamino-1-(4-(3,4-dimethoxy-phenyl)aminomethyl-3-thienyl)-benzol, 2,5-Diamino-1-(4-(4-trifluormethyl-benzyl)aminomethyl-3-thienyl)-benzol, 2,5-Diamino-1-(4-(3-methoxy-phenyl)aminomethyl-3-thienyl)-benzol, 2,5-Diamino-1-(4-(O,N-dimethyl-hydroxylaminomethyl-3-thienyl)-benzol, 2,5-Diamino-1-(4-(2,4-dimethoxy-5-chlor-phenyl)aminomethyl-3-thienyl)-benzol, 2,5-Diamino-1-(4-(4-phenoxy-phenyl)aminomethyl-3-thienyl)-benzol, 2,5-Diamino-1-(4-(2-diphenyl)aminomethyl-3-thienyl)-benzol, 2,5-Diamino-1-(4-(3,4-dimethoxybenzyl)aminomethyl-3-thienyl)benzol, 2,5-Diamino-1-(4-(4-methoxyphenyl)aminomethyl-3-thienyl)-benzol, 2,5-Diamino-1-(4-(4-hydroxy-butyl)aminomethyl-3-thienyl)-benzol, 2,5-Diamino-1-(4-(2-methoxy-3-fluor-phenyl)aminomethyl-3-thienyl)-benzol, 2,5-Diamino-1-(4-(4-(1-phenyl-ethyl)aminomethyl-3-thienyl)-benzol, 2,5-Diamino-1-(4-(2-furyl)aminomethyl-3-thienyl)-benzol, 2,5-Diamino-1-(4-(2-pyridyl)aminomethyl-3-thienyl)-benzol, 2,5-Diamino-1-(4-morpholinomethyl-3-thienyl)-benzol, 2,5-Diamino-1-(4-4-benzyl-pyperazinomethyl-3-thienyl)-benzol, 2,5-Diamino-1-(4-(3-carboxamid-phenyl)aminomethyl-3-thienyl)-benzol, 2,5-Diamino-1-(4-(N-methyl-N-phenyl)aminomethyl-3-thienyl)-benzol, 2,5-Diamino-1-(4-diethylaminomethyl-3-thienyl)-benzol, 2,5-Diamino-1-(4-(pyridin-4yl-methyl)aminomethyl-3-thienyl)-benzol, 2,5-Diamino-1-(4-(3,5-dimethoxy-benzyl)aminomethyl-3-thienyl)-benzol, 2,5-Diamino-1-(4-(4-morpholino-phenyl)aminomethyl-3-thienyl)-benzol, 2,5-Diamino-1-(4-(1-(2-hydroxyethyl))propylaminomethyl-3-thienyl)-benzol, 2,5-Diamino-1-(4-cyclopropylaminomethyl-3-thienyl)-benzol, N-(2-{[4-(2,5-Diamino-phenyl)-thiophen-2-ylmethyl]-amino}-ethyl)-acetamid, 2,5-Diamino-1-(4-cyclohexylaminomethyl-3-thienyl)-benzol, 2,5-Diamino-1-(4-propylaminomethyl-3-thienyl)-benzol, 2,5-Diamino-1-(4-(o-tolyl)aminomethyl-3-thienyl)-benzol, 2,5-Diamino-1-(4-(N-methyl-N-cyclohexyl)aminomethyl-3-thienyl)-benzol, 2,5-Diamino-1-(4-(2,6-dimethyl-morpholino)methyl-3-thienyl)-benzol, 2,5-Diamino-1-(4-(3,5-dimethyl-piperidino)methyl-3-thienyl)-benzol, 2,5-Diamino-1-(4-(3-(1-hydroxyethyl)phenyl)aminomethyl-3-thienyl)-benzol, 2,5-Diamino-1-(4-(3,4-dimethyt-phenyl)aminomethyl-3-thienyl)-benzol, 2,5-Diamino-1-(4-(3-methylmercapto-phenyl)aminomethyl-3-thienyl)-benzol, 2,5-Diamino-1-(4-(4-diphenyl)aminomethyl-3-thienyl)-benzol, 2,5-Diamino-1-(4-(4-isopropyl-phenyl)aminomethyl-3-thienyl)-benzol, 2,5-Diamino-1-(4-pentylaminomethyl-3-thienyl)-benzol, 2,5-Diamino-1-(4-(dibutyl)aminomethyl-3-thienyl)-benzol, 2,5-Diamino-1-(4-isopropylaminomethyl-3-thienyl)-benzol, 2,5-Diamino-1-(4-(N-Cyclopropylmethyl-N-propyl)aminomethyl-3-thienyl)-benzol, 2,5-Diamino-1-(4-(4-hydroxy)piperidinomethyl-3-thienyl)-benzol, 2,5-Diamino-1-(4-(fluorenyl)aminomethyl-3-thienyl)-benzol, 2,5-Diamino-1-(4-(4-Dimethylamino-phenyl)aminomethyl-3-thienyl)-benzol, 2,5-Diamino-1-(4-(3,5-dimethyl-phenyl)aminomethyl-3-thienyl)-benzol, 2,5-Diamino-1-(4-(4-methoxy-benzyl)aminomethyl-3-thienyl)-benzol, 2,5-Diamino-1-(4-(indanyl)aminomethyl-3-thienyl)-benzol, 2,5-Diamino-1-(4-(3-fluor-benzyl)aminomethyl-3-thienyl)-benzol, 2,5-Diamino-1-(4-(benzyl)aminomethyl-3-thienyl)-benzol, 2,5-Diamino-1-(4-(4-methyl-piperazino)aminomethyl-3-thienyl)-benzol, 2,5-Diamino-1-(4-(4-tert-butyl-phenyl)aminomethyl-3-thienyl)-benzol, 2,5-Diamino-1-(4-(2-chlor-benzyl)aminomethyl-3-thienyl)-benzol, 2,5-Diamino-1-(4-(napht-1-yl-methyl)aminomethyl-3-thienyl)-benzol, 2,5-Diamino-1-(4-(phenyl)aminomethyl-3-thienyl)-benzol, 2,5-Diamino-1-(4-(4-chlor-phenyl)aminomethyl-3-thienyl)benzol, 4-{[4-(2,5-Diamino-phenyl)-thiophen-2-ylmethyl]-amino}-benzosäuremethyl-ester und 2,5-Diamino-1-(3-thienyl)benzol, wobei das 2,5-Diamino-1-(3-thienyl)benzol besonders bevorzugt ist.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die Herstellung der erfindungsgemäßen Diaminobenzol-Derivate der Formel (I) kann unter Verwendung von bekannten Syntheseverfahren erfolgen. Die Synthese der erfindungsgemäßen Verbindungen kann beispielsweise wie folgt durchgeführt werden:
Entweder a) durch eine Tetrakis(triphenylphospin)palladium(0) katalysierte Kupplung eines substituierten Benzols der Formel (II) 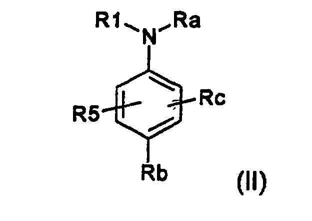 mit einer Heteroarylverbindung der Formel (III) 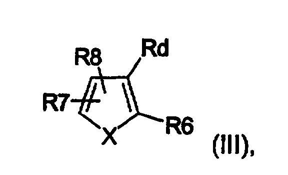 worin
   Ra die Bedeutung einer Schutzgruppe, wie sie zum Beispiel in dem Kapitel "Protective Groups" in Organic Synthesis, Kapitel 7, Wiley Interscience, 1991 beschrieben wird,
   Rb die Bedeutung NR1Ra oder NO₂ hat,
   Rc die Bedeutung Halogen und Rd die Bedeutung B(OH)₂ beziehungsweise Rc die Bedeutung B(OH)₂ und Rd die Bedeutung Halogen hat, und
   X, R1, R5, R6, R7 und R8 die in Formel (I) gennante Bedeutung haben, und anschließende Abspaltung der Schutzgruppe oder Abspaltung der Schutzgruppe und Reduktion der Nitrogruppe;
oder b) durch eine Tetrakis(triphenylphospin)palldium(0) katalysierte Kupplung eines substituierten Benzols der Formel (IV) 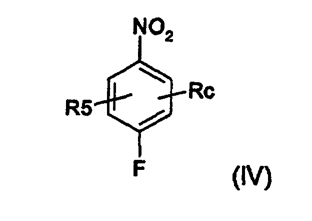 mit einer Heteroarylverbindung der Formel (III) 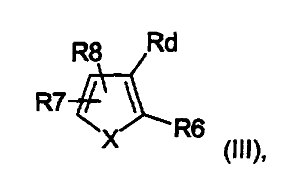 worin
   Rc die Bedeutung Halogen und Rd die Bedeutung B(OH)₂ beziehungsweise Rc die Bedeutung B(OH)₂ und Rd die Bedeutung Halogen hat, und
   X, R5, R6, R7 und R8 die in Formel (I) angegebene Bedeutung haben, anschließende Substitution des so erhaltenen substituierten Benzols der Formel (V) 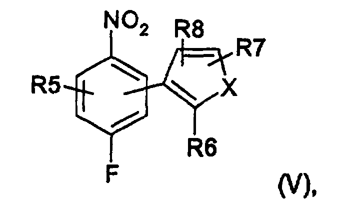 mit einem Amin der Formel HNR1R2,
   worin R1,R2 die in Formel (I) genannte Bedeutung haben, und Reduktion der Nitrogruppe.

Die erfindungsgemäßen Diaminobenzol-Derivate der Formel (I) sind in Wasser gut löslich und ermöglichen Färbungen mit hoher Farbintensität und ausgezeichneter Farbechtheit, insbesondere was die Lichtechtheit, Waschechtheit und Reibeechtheit anbetrifft. Die Verbindungen der Formen (I) weisen weiterhin eine ausgezeichnete Lagerstabilität, insbesondere als Bestandteil der nachfolgend beschriebenen Färbemittel, auf.

Ein weiterer Gegenstand der vorliegenden Erfindung sind daher Mittel zum oxidativen Färben von Keratinfasern, wie zum Beispiel Haaren, Pelzen, Federn oder Wolle, insbesondere menschlichen Haaren, auf der Basis einer Entwicklersubstanz-Kupplersubstanz-Kombination, welche als Entwicklersubstanz mindestens ein Diaminobenzol-Derivat der Formel (I) enthalten.

Das Diaminobenzol-Derivat der Formel (I) ist in dem erfindungsgemäßen Färbemittel in einer Menge von etwa 0,005 bis 20 Gewichtsprozent enthalten, wobei eine Menge von etwa 0,01 bis 5,0 Gewichtsprozent und insbesondere 0,1 bis 2,5 Gewichtsprozent bevorzugt ist.

Als Kupplersubstanzen kommen vorzugsweise 2,6-Diamino-pyridin, 2-Amino-4-[(2-hydroxyethyl)amino]-anisol, 2,4-Diamino-1-fluor-5-methylbenzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxypyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diaminobenzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxyessigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlorphenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxynaphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxybenzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxyphenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxyindol,7-Hydroxy-indol und 2,3-Indolindion in Betracht.

Obwohl die vorteilhaften Eigenschaften der hier beschriebenen Diaminobenzol-Derivate der Formel (I) es nahelegen, diese als alleinige Entwicklersubstanz zu verwenden, ist es selbstverständlich auch möglich, die Diaminobenzol-Derivate der Formel (I) gemeinsam mit bekannten Entwicklersubstanzen, wie zum Beispiel 1,4-Diaminobenzol, 2,5-Diaminotoluol, 2,5-Diaminophenylethylalkohol, 4-Aminophenol und seinen Derivaten, beispielsweise 4-Amino-3-methylphenol, 4,5-Diamino-1-(2-hydroxyethyl)-pyrazol oder Tetraaminopyrimidinen, einzusetzen.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5,0 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6,0 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Uberschuß oder Unterschuß vorhanden sind.

Weiterhin kann das erfindungsgemäße Färbemittel zusätzlich andere Farbkomponenten, beispielsweise 6-Amino-2-methylphenol und 2-Amino-5-methylphenol, sowie ferner übliche direktziehende Farbstoffe, zum Beispiel Triphenylmethanfarbstoffe wie 4-[(4'-aminophenyl)-(4'imino-2",5"-cyclohexadien-1"-yliden)-methyl]-2-methylaminobenzol-monohydrochlorid (C.I. 42 510) und 4-[(4'amino-3'-methyl-phenyl)-(4"-imino-3"-methyl-2",5"cyclohexadien-1"-yliden)-methyl]-2-methyl-aminobenzol monohydrochlorid (C.I. 42 520), aromatische Nitrofarbstoffe wie 4-(2'-hydroxyethyl)amino-nitrotoluol, 2-Amino-4,6-dinitrophenol, 2-Amino-5-(2'-hydroxyethyl)amino-nitrobenzol, 2-Chlor-6-(ethylamino)-4-nitrophenol, 4-Chlor-N-(2-hydroxyethyl)-2-nitroanllin, 5-Chlor-2-hydroxy-4-nitroanilin, 2-Amino-4-chlor-6-nitrophenol und 1-[(2'-Ureidoethyl)amino-4-nikobenzol, Azofarbstoffe wie 6-[(4'-Aminophenyl)azo]-5hydroxy-naphthalin-1-sulfonsäure-Natriumsalz (C.I. 14 805) und Dispersionsfarbstoffe wie beispielsweise 1,4-Diaminoanthrachinon und 1,4,5,8-Tetraaminoantrachinon, enthalten. Die Färbemittel können diese Farbkomponenten in einer Menge von etwa 0,1 bis 4,0 Gewichtsprozent enthalten.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein. Die Zubereitungsform des erfindungsgemäßen Färbemittels kann beispielsweise eine Lösung, insbesondere eine wäßrige oder wäßrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent

Je nach Zusammensetzung kann das erfindungsgemäße Färbemittel schwach sauer, neutral oder alkalisch reagieren. Insbesondere weist es einen pH-Wert von 6,8 bis 11,5 auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet

Die erfindungsgemäßen Haarfärbemittel mit einem Gehalt an Diaminobenzol-Derivaten der Formel (I) als Entwicklersubstanz ermöglichen Haarfärbungen mit ausgezeichneter Farbechtheit, insbesondere was die Lichtechtheit, Waschechtheit und Reibeechtheit anbetrifft. Hinsichtlich der färberischen Eigenschaften bieten die erfindungsgemäßen Haarfärbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt Hierbei zeichnen sich die Farbtöne durch ihre besondere Farbintensität aus. Die sehr guten färberischen Eigenschaften der Haarfärbemittel gemäß der vorliegenden Anmeldung zeigen sich weiterhin darin, daß diese Mittel eine Anfärbung von ergrauten, chemisch nicht vorgeschädigten Haaren problemlos und mit guter Deckkraft ermöglichen.

Die nachfolgenden Beispiele sollen den Gegenstand der Erfindung näher erläutern, ohne ihn darauf zu beschränken.

### Beispiele

### Beispiele 1: Synthese von 2,5-Diamino-1-(3-thienyl)-benzolen (Allgemeine Synthesevorschrift)

### A. Synthese von 2,5-tert.-Butyloxycarbonylamino-brombenzol

15,65 g (0,07 mol) Brom-p-phenylendiamin-Hydrochlorid und 32,7 g (0,15 mol) Di-tert.-butyl-dicarbonat werden in einer Mischung von 250 ml
2N Natriunhydroxide und 250 ml Trifluortoluol gelöst und auf 45 °C erwärmt. Die Reaktionmischung wird 3 Tage gerührt. Schrittweise werden noch insgesamt 30 g (0,14 mol) Di-tert.-butyl-dicarbonat zugegeben. Anschließend wird die organische Schicht abgetrennt und die wäßrige Phase noch zweimal mit 100 ml Dichlormethan extrahiert. Die vereinigten Extrakte weren eingedampft und der Rückstand in 200 ml Hexan aufgenommen. Der Niederschlag wird abfiltriert und mit 50 ml Hexan nachgewaschen.
Es werden 18,6 g (82 % der Theorie) 2,5-tert.-Butyloxycarbonylamino-brombenzol mit einem Schmelzpunkt von 130 °C erhalten.

### B. Synthese von 2,5-Diamino-1-(3-thienyl)-benzolen

3,3 g (0,01 mol) 2,5-tert.-Butyloxycarbonylamino-brombenzol aus Stufe **A** und 0,013 mol der entsprechenden Borsäure werden unter Argon in 70 ml 1,2-Dimethoxyethan gelöst. Anschließend werden 0,5 g Tetrakis-(triphenylphosphin)-palladium (0,0005 mol) und 13 ml 2N Kaliumcarbonat-lösung zugegeben und die Reaktionsmischung auf 80 °C erwärmt. Nach Beendigung der Reaktion wird die Reaktionsmischung in 100 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt Das so erhaltene Produkt wird in 40 ml Ethanol auf 50 °C erwärmt.
Anschließend werden zur Herstellung des Hydrochlorides 15 ml einer 2,9 molaren ethanolischen Salzsäurelösung zugetropft. Der Niederschlag wird abfiltriert, zweimal mit 10 ml Ethanol gewaschen und sodann getrocknet.

### 2,5-Diamino-1-(3-thienyl)benzol-dihydrochlorid

Verwendete Borsäure: Thiophen-3-borsäure
Ausbeute: 2,0 g (75 % der Theorie)
Schmelzpunkt: 245 °Celsius (farblose Kristalle)

| CHN-Analyse: | | | |
|---|---|---|---|
| (C₁₀H₁₂N₂Cl₂S) | % C | % H | % N |
| berechnet | 45,64 | 4,60 | 10,64 |
| gefunden | 45,55 | 4,66 | 10,64 |

### C. Synthese von N,N'-Bis(tert-Butoxycarbonyl)-2,5-diamino-1-phenylborsäure

Die N,N'-Bis(tert-Butoxycarbonyl)-2,5-diamino-1-phenylborsäure werden durch Umsetzung von N,N'-Bis(tert-Butoxycarbonyl)-2,5-diamino-1-brombenzol mit tert-Butyllithium und Trimethylborate dargestellt Die experimentelle Vorschrift dieser Herstellungsmethode wird von J. M. Tour und J. J. S: Lamba in J. Am. Chem. Soc.1994,116 Seite 11723 beschrieben.

### D. Synthese von 2,5-Diamino-1-(3-thienyl)-benzolen und 2,5-Diamino-1-(3-furyl)-benzol

0,035 g (0,0001 mol) 2,5-tert.-Butyloxycarbonylamino-1-phenylborsäure aus Stufe **C** und 0,00015 mol des entsprechenden Bromderivates werden unter Argon in 10 ml 1,2-Dimethoxyethan gelöst. Anschließend werden 0,005 g Tetrakis-(triphenylphosphin)-palladium (0,000005 mol) und 0,13 ml 2N Kaliumcarbonat-lösung zugegeben und die Reaktionsmischung auf 80 °C erwärmt. Nach Beendigung der Reaktion wird die Reaktionsmischung in 10 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol auf 50 °C erwärmt.
Anschließend werden zur Herstellung des Hydrochlorides 1,5 ml einer 2,9 molaren ethanolische Salzsäurelösung zugetropft. Der Niederschlag wird abfiltriert, zweimal mit 1 ml Ethanol gewaschen und sodann getrocknet.

### a. 2,5-Diamino-1-(5-methyl-3-thienyl)benzol-dihydrochlorid

Verwendete Bromderivat: 3-Brom-4-methylthiophen
Ausbeute: 0.025 g (90 % der Theorie)
Masspektren M⁺ 205 (100)

### b. 2,5-Diamino-1-(2-chlor-3-thienyl)benzol-dihydrochlorid

Verwendete Bromderivat: 3-Brom-2-chlorthiophen
Ausbeute: 0.025 g (84 % der Theorie)
Masspektren M⁺ 225 (100)

### c. 2,5-Diamino-1-(4-(4-nitropropenyl)-3-thienyl)benzol-dihydrochlorid

Verwendete Bromderivat: 3-Brom-5-(2-nitropropenyl)thiophen
Ausbeute: 0.025 g ( 70 % der Theorie)
Masspektren MH⁺ 276 (100)

### d. 2,5-Diamino-1-(3-furyl)benzol-dihydrochlorid

Verwendete Bromderivat: 3-Brom-furan
Ausbeute: 0.025 g ( 90 % der Theorie)
Masspektren MH⁺ 175 (100)

### e. 2,5-Diamino-1-(4-formyl-3-thienyl)benzol-dihydrochlorid

Verwendete Bromderivat: 3-Brom-5-formylthiophen
Ausbeute: 0.025 g (71 % der Theorie)
Masspektren MH⁺ 219 (100)

### E. Synthese von 2,5-Diamino-1-(4-aminomethyl3-thienyl)-benzolen

0,030 g (0,0001 mol) 2,5-tert.-Butyloxycarbonylamino-1-(4-(4-formyl-3-thienyl)benzol aus Stufe **D.e** und 0,00015 mol des entsprechenden Amins werden in Methanol gelöst und mit NaBH4 reduziert.
Nach Beendigung der Reaktion wird die Reaktionsmischung in 10 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol auf 50 °C erwärmt.
Anschließend werden zur Herstellung des Hydrochlorides 1,5 ml einer 2,9 molaren ethanolische Salzsäurelösung zugetropft. Der Niederschlag wird abfiltriert, zweimal mit 1 ml Ethanol gewaschen und sodann getrocknet.

### a. 2,5-Diamino-1-(4-(2-hydroxyethyl)aminomethyl-3-thienyl)-benzol-dihydrochlorid

Verwendetes Amin: Ethanolamin
Ausbeute: 0.025 g (67 % der Theorie)
Masspektren M⁺ 264 (100)

### b. 2.5-Diamino-1-(4-pyrrolidinomethyl-3-thienyl)-benzol-dihydrochlorid

Verwendetes Amin: Pyrrolidin
Ausbeute: 0.025 g (65 % der Theorie)
Masspektren MH⁺ 274 (100)

### c. 2,5-Diamino-1-(4-(dimethylaminoethyl)aminomethyl-3-thienyl)-benzol-dihydrochlorid

Verwendetes Amin: Dimethylaminoethylamin
Ausbeute: 0.025 g ( 57 % der Theorie)
Masspektren M⁺ 291(100)

### d. 2,5-Diamino-1-(4-(2-methoxy-5-chlor-phenyl)aminomethyl-3-thienyl)-benzol-dihydrochlorid

Verwendetes Amin: 2-Methoxy-5-chloranilin
Ausbeute: 0.025 g ( 53 % der Theorie)
Masspektren MH⁺ 360 (100)

### e. 2,5-Diamino-1-(4-(3-ethoxypropyl)aminomethyl-3-thienyl)-benzol-dihydrochlorid

Verwendetes Amin: 3-Ethoxypropylamin
Ausbeute: 0.025 g (60 % der Theorie)
Masspektren MH⁺ 306 (100)

### f. 2,5-Diamino-1-(4-(methoxyethyl)aminomethyl-3-thienyl)-benzol-dihydrochlorid

Verwendetes Amin: Methoxyethylamin
Ausbeute: 0.025 g (64 % der Theorie)
Masspektren MH⁺ 278 (100)

### g. 2,5-Diamino-1-(4-(3,4-dimethoxy-phenyl)aminomethyl-3-thienyl)-benzol-dihydrochlorid

Verwendetes Amin: 3,4-Dimethoxyanilin
Ausbeute: 0.025 g ( 54 % der Theorie)
Masspektren MH⁺ 356 (100)

### h. 2,5-Diamino-1-(4-(4-trifluormethyl-benzyl)aminomethyl-3-thienyl)-benzol-dihydrochlorid

Verwendetes Amin: 4-Trifluormethyl-benzylamin
Ausbeute: 0.025 g (54 % der Theorie)
Masspektren MH⁺ 378 (100)

### i. 2,5-Diamino-1-(4-(3-methoxy-phenyl)aminomethyl-3-thienyl)-benzol-dihydrochlorid

Verwendetes Amin: 3-Methoxyanilin
Ausbeute: 0.025 g (57 % der Theorie)
Masspektren MH⁺ 326 (100)

### j. 2,5-Diamino-1-(4-(O,N-dimethyl-hydroxylaminomethyl-3-thienyl)-benzol-dihydrochlorid

Verwendetes Amin: O,N-Dimethylhydroxylamin
Ausbeute: 0.025 g ( 67 % der Theorie)
Masspektren MH⁺ 264 (100)

### k. 2,5-Diamino-1-(4-(2,4-dimethoxy-5-chlor-phenyl)aminomethyl-3-thienyl)-benzol-dihydrochlorid

Verwendetes Amin: 2,4-Dimetoxy-5-chloranilin
Ausbeute: 0.025 g (50 % der Theorie)
Masspektren MH⁺ 390 (100)

### I. 2,5-Diamino-1-(4-(4-phenoxy-phenyl)aminomethyl-3-thienyl)-benzol-dihydrochlorid

Verwendetes Amin: 4-Phenoxyanilin
Ausbeute: 0.025 g (50 % der Theorie)
Masspektren MH⁺ 388 (100)

### m. 2,5-Diamino-1-(4-(2-diphenyl)aminomethyl-3-thienyl)-benzol-dihydrochlorid

Verwendetes Amin: 2-Diphenylamin
Ausbeute: 0.025 g (52 % der Theorie)
Masspektren MH⁺ 372 (100)

### n. 2,5-Diamino-1-(4-(3,4-dimethoxybenzyl)aminomethyl-3-thienyl)-benzol-dihydrochlorid

Verwendetes Amin: 3,4-Dimethoxybenzylamin
Ausbeute: 0.025 g (52 % der Theorie)
Masspektren MH⁺ 370 (100)

### o. 2,5-Diamino-1-(4-(4-methoxyphenyl)aminomethyl-3-thienyl)-benzol-dihydrochlorid

Verwendetes Amin: 4-Methoxyanilin
Ausbeute: 0.025 g (50 % der Theorie)
Masspektren MH⁺ 326 (100)

### p. 2,5-Diamino-1-(4-(4-hydroxy-butyl)aminomethyl-3-thienyl)-benzol-dihydrochlorid

Verwendetes Amin: 4-Hydroxy-butylamin
Ausbeute: 0.025 g (62 % der Theorie)
Masspektren MH⁺ 292(100)

### q. 2,5-Diamino-1-(4-(2-methoxy-3-fluor-phenyl)aminomethyl-3-thienyl)-benzol-dihydrochlorid

Verwendetes Amin: 4-Methoxy-3-fluor-anilin
Ausbeute: 0.025 g (55 % der Theorie)
Masspektren MH⁺ 344 (100)

### r. 2,5-Diamino-1-(4-(4-(1-phenyl-ethyl)aminomethyl-3-thienyl)-benzol-dihydrochlorid

Verwendetes Amin: 1-Phenyl-ethylamin
Ausbeute: 0.025 g (58 % der Theorie)
Masspektren MH⁺ 324(100)

### s. 2,5-Diamino-1-(4-(2-furyl)aminomethyl-3-thienyl)-benzol-dihydrochlorid

Verwendetes Amin: 2-Furylamin
Ausbeute: 0.025 g (61 % der Theorie)
Masspektren MH⁺ 300(100)

### t. 2,5-Diamino-1-(4-(2-pyridyl)aminomethyl-3-thienyl)-benzol-dihydrochlorid

Verwendetes Amin: 2-Aminopyridin
Ausbeute: 0.025 g (56 % der Theorie)
Masspektren MH⁺ 297 (100)

### u. 2,5-Diamino-1-(4-morpholinomethyl-3-thienyl)-benzol-dihydrochlorid

Verwendetes Amin: Morpholin
Ausbeute: 0.025 g ( 63 % der Theorie)
Masspektren MH⁺ 290 (100)

### v. 2,5-Diamino-1-(4-4-benzyl-pyperazinomethyl-3-thienyl)-benzol-dihydrochlorid

Verwendetes Amin: 4-Benzyl-pyperazin
Ausbeute: 0.025 g ( 47 % der Theorie)
Masspektren MH⁺ 379 (100)

### w. 2,5-Diamino-1-(4-(3-carboxamid-phenyl)aminomethyl-3-thienyl)-benzol-dihydrochlorid

Verwendetes Amin: 3-Amino-benzamid
Ausbeute: 0.025 g ( 56 % der Theorie)
Masspektren MH⁺ 339(100)

### x. 2,5-Diamino-1-(4-(N-methyl-N-phenyl)aminomethyl-3-thienyl)-benzol-dihydrochlorid

Verwendetes Amin: N-methyl-N-phenylamin
Ausbeute: 0.025 g (59 % der Theorie)
Masspektren MH⁺ 310(100)

### y. 2,5-Diamino-1-(4-diethylaminomethyl-3-thienyl)-benzol-dihydrochlorid

Verwendetes Amin: Diethylamin
Ausbeute: 0.025 g (65 % der Theorie)
Masspektren MH⁺ 276 (100)

### z. 2,5-Diamino-1-(4-(pyridin-4yl-methyl)aminomethyl-3-thienyl)-benzol-dihydrochlorid

Verwendetes Amin: 4-Aminomethyl-pyridin
Ausbeute: 0.025 g (55 % der Theorie)
Masspektren MH⁺ 311 (100)

### a'. 2,5-Diamino-1-(4-(3,5-dimethoxy-benzyl)aminomethyl-3-thienyl)-benzol-dihydrochlorid

Verwendetes Amin: 3,5-Dimethoxy-benzylamin
Ausbeute: 0.025 g (52 % der Theorie)
Masspektren MH⁺ 370 (100)

### b'. 2,5-Diamino-1-(4-(4-morpholino-phenyl)aminomethyl-3-thienyl)-benzol-dihydrochlorid

Verwendetes Amin: 4-Morpholinoanilin
Ausbeute: 0.025 g (47 % der Theorie)
Masspektren MH⁺ 381 (100)

### c'. 2,5-Diamino-1-(4-(1-(2-hydroxyethyl))propylaminomethyl-3-thienyl)-benzol-dihydrochlorid

Verwendetes Amin: 1-(2-Hydroxyethyl)propylamin
Ausbeute: 0.025 g (62 % der Theorie)
Masspektren MH⁺ 292 (100)

### d'. 2,5-Diamino-1-(4-cyclopropylaminomethyl-3-thienyl)-benzol-dihydrochlorid

Verwendetes Amin: Cyclopropylamin
Ausbeute: 0.025 g (68 % der Theorie)
Masspektren MH⁺ 260(100)

### e'. N-(2-{[4-(2,5-Diamino-phenyl)-thiophen-2-ylmethyl]-amino}-ethyl)-acetamide-dihydrochlorid

Verwendetes Amin: 2-Aminoethylacetamid
Ausbeute: 0.025 g (60 % der Theorie)
Masspektren MH⁺ 305 (100)

### f'. 2,5-Diamino-1-(4-cyclohexylaminomethyl-3-thienyl)-benzol-dihydrochlorid

Verwendetes Amin: Cyclohexylamin
Ausbeute: 0.025 g (60 % der Theorie)
Masspektren MH⁺ 302(100)

### g'. 2,5-Diamino-1-(4-propylaminomethyl-3-thienyl)-benzol-dihydrochlorid

Verwendetes Amin: propylamin
Ausbeute: 0.025 g (67 % der Theorie)
Masspektren MH⁺ 262 (100)

### h'. 2,5-Diamino-1-(4-(o-tolyl)aminomethyl-3-thienyl)-benzol-dihydrochlorid

Verwendetes Amin: 2-Methylanilin
Ausbeute: 0.025 g (59 % der Theorie)
Masspektren MH⁺ 310(100)

### i'. 2,5-Diamino-1-(4-(N-methyl-N-cyclohexyl)aminomethyl-3-thienyl) benzol-dihydrochlorid

Verwendetes Amin: N-methyl-N-cyclohexylamin
Ausbeute: 0.025 g (59 % der Theorie)
Masspektren MH⁺ 316 (100)

### j'. 2,5-Diamino-1-(4-(2,6-dimethyl-morpholino)methyl-3-thienyl)-benzol-dihydrochlorid

Verwendetes Amin: 2.6-Dimethyl-morpholin
Ausbeute: 0.025 g (58 % der Theorie)
Masspektren MH⁺ 318 (100)

### k'. 2,5-Diamino-1-(4-(3,5-dimethyl-piperidino)methyl-3-thienyl)-benzol-dihydrochlorid

Verwendetes Amin: 3,5-Dimethyl-piperidin
Ausbeute: 0.025 g (59 % der Theorie)
Masspektren MH⁺ 340 (100)

### l'. 2,5-Diamino-1-(4-(3-(1-hydroxyethyl)phenyl)aminomethyl-3-thienyl)-benzol-dihydrochlorid

Verwendetes Amin: 3-(1-Hydroxyethyl)anilin
Ausbeute: 0.025 g ( 55 % der Theorie)
Masspektren MH⁺ 340 (100)

### m'. 2,5-Diamino-1-(4-(3,4-dimethyl-phenyl)aminomethyl-3-thienyl)-benzol-dihydrochlorid

Verwendetes Amin: 3,4-Dimethyl-anilin
Ausbeute: 0.025 g (58 % der Theorie)
Masspektren MH⁺ 324 (100)

### n'. 2,5-Diamino-1-(4-(3-methylmercapto-phenyl)aminomethyl-3-thienyl)-benzol-dihydrochlorid

Verwendetes Amin: 3-methylmercaptoanilin
Ausbeute: 0.025 g (55 % der Theorie)
Masspektren MH⁺ 342 (100)

### o'. 2,5-Diamino-1-(4-(4-diphenyl)aminomethyl-3-thienyl)-benzol-dihydrochlorid

Verwendetes Amin: 4-Amino-biphenyl
Ausbeute: 0.025 g (52 % der Theorie)
Masspektren MH⁺ 372 (100)

### p'. 2,5-Diamino-1-(4-(4-isopropyl-phenyl)aminomethyl-3-thienyl)-benzol-dihydrochlorid

Verwendetes Amin: 4-Isopropyl-anilin
Ausbeute: 0.025 g (56 % der Theorie)
Masspektren MH⁺ 338 (100)

### q'. 2,5-Diamino-1-(4-pentylaminomethyl-3-thienyl)-benzol-dihydrochlorid

Verwendetes Amin: Pentylamin
Ausbeute: 0.025 g ( 63 % der Theorie)
Masspektren MH⁺ 290(100)

### r'. 2,5-Diamino-1-(4-(dibutyl)aminomethyl-3-thienyl)-benzol-dlhydrochlorid

Verwendetes Amin: Dibutylamin
Ausbeute: 0.025 g (57 % der Theorie)
Masspektren MH⁺ 332(100)

### s'. 2,5-Diamino-1-(4-isopropylaminomethyl-3-thienyl)-benzol-dihydrochlorid

Verwendetes Amin: Isopropylamin
Ausbeute: 0.025 g (67 % der Theorie)
Masspektren MH⁺ 262(100)

### t'. 2,5-Diamino-1-(4-N-Cyclopropylmethyl-N-propyl)aminomethyl-3-thienyl)-benzol-dihydrochlorid

Verwendetes Amin: N-Cyclopropylmethyl-propylamin
Ausbeute: 0.025 g (59 % der Theorie)
Masspektren MH⁺ 316 (100)

### u'. 2,5-Diamino-1-(4-(4-hydroxy)piperidinomethyl-3-thienyl)-benzol-dihydrochlorid

Verwendetes Amin: 4-Hydroxy-piperidin
Ausbeute: 0.025 g (60 % der Theorie)
Masspektren MH⁺ 304(100)

### v'. 2,5-Diamino-1-(4-(fluorenyl)aminomethyl-3-thienyl)-benzol-dihydrochlorid

Verwendetes Amin: 2-Amino-fluoren
Ausbeute: 0.025 g ( 51 % der Theorie)
Masspektren MH⁺ 384(100)

### w'. 2,5-Diamino-1-(4-(4-Dimethylamino-phenyl)aminomethyl-3-thienyl)-benzol-dihydrochlorid

Verwendetes Amin: 4-Dimethylamino-anilin
Ausbeute: 0.025 g (52 % der Theorie)
Masspektren MH⁺ 339 (100)

### x'. 2,5-Diamino-1-(4-(3,5-dimethyl-phenyl)aminomethyl-3-thienyl)-benzol-dihydrochlorid

Verwendetes Amin: 3,5-Dimethyl-anilin
Ausbeute: 0.025 g (58 % der Theorie)
Masspektren MH⁺ 324 (100)

### y'. 2,5-Diamino-1-(4-(4-methoxy-benzyl)aminomethyl-3-thienyl)benzol-dihydrochlorid

Verwendetes Amin: 4-methoxy-benzylamin
Ausbeute: 0.025 g (55 % der Theorie)
Masspektren MH⁺ 340 (100)

### z'. 2,5-Diamino-1-(4-(indanyl)aminomethyl-3-thienyl)-benzol-dihydrochlorid

Verwendetes Amin: 1-Amino-indan
Ausbeute: 0.025 g (56 % der Theorie)
Masspektren MH⁺ 336 (100)

### a". 2,5-Diamino-1-(4-(3-fluor-benzyl)aminomethyl-3-thienyl)-benzol-dihydrochlorid

Verwendetes Amin: 3-Fluor-benzylamin
Ausbeute: 0.025 g (57 % der Theorie)
Masspektren MH⁺ 328 (100)

### b". 2,5-Diamino-1-(4-(benzyl)aminomethyl-3-thienyl)benzol-dihydrochlorid

Verwendetes Amin: Benzylamin
Ausbeute: 0.025 g (60 % der Theorie)
Masspektren MH⁺ 310 (100)

### c". 2,5-Diamino-1-(4-(4-methyl-piperazino)aminomethyl-3-thienyl)-benzol-dihydrochlorid

Verwendetes Amin: 4-Methylpiperazin
Ausbeute: 0.025 g (56 % der Theorie)
Masspektren MH⁺ 303 (100)

### d". 2,5-Diamino-1-(4-(4-tert-butyl-phenyl)aminomethyl-3-thienyl)-benzol-dihydrochlorid

Verwendetes Amin: 4-tert-Butylanilin
Ausbeute: 0.025 g (54 % der Theorie)
Masspektren MH⁺ 352 (100)

### e". 2,5-Diamino-1-(4-(2-chlor-benzyl)aminomethyl-3-thienyl)-benzol-dihydrochlorid

Verwendetes Amin: 2-Chlor-benzylamin
Ausbeute: 0.025 g (55 % der Theorie)
Masspektren MH⁺ 344 (100)

### f". 2,5-Diamino-1-(4-(napht-1-yl-methyl)aminomethyl-3-thienyl)-benzol-dihydrochlorid

Verwendetes Amin: 1-Aminomethylnaphtalen
Ausbeute: 0.025 g (53 % der Theorie)
Masspektren MH⁺ 360 (100)

### g". 2,5-Diamino-1-(4-(phenyl)aminomethyl-3-thienyl)-benzol-dihydrochlorid

Verwendetes Amin: Anilin
Ausbeute: 0.025 g ( 62 % der Theorie)
Masspektren MH⁺ 296(100)

### h". 2,5-Diamino-1-(4-(4-chlor-phenyl)aminomethyl-3-thienyl)-benzol-dihydrochlorid

Verwendetes Amin: 4-Chlor-anilin
Ausbeute: 0.025 g (57 % der Theorie)
Masspektren MH⁺ 330 (100)

### i". 4-{[4-(2,5-Diamino-phenyl)-thiophen-2-ylmethyl]-amino}-benzosäure-methyl-ester dihydrochlorid

Verwendetes Amin: 4-Amino-benzosäure-methylester
Ausbeute: 0.025 g (54 % der Theorie)
Masspekken MH⁺ 354 (100)

### Beispiel 2: Synthese von 2-N-substituierten 2-Amino-5-amino-1-(3-thienyl)-benzolen (Allgemeine Synthesevorschrift)

### A. Synthese von 2-Fluor-5-nitro-1-(3-thienyl)-benzol

1,75 g (0,01 mol) 1-Chlor-2-fluoro-5-nitrobenzol und 0,013 mol Thiophen-3-borsäure werden unter Argon in 70 ml 1,2-Dimethoxyethan gelöst. Anschließend werden 0,5 g Tetrakis-(triphenylphosphin)-palladium (0,0005 mol) und 13 ml 2N Kaliumcarbonat-Iösung zugegeben und die Reaktionsmischung auf 80 °C erwärmt. Nach Beendigung der Reaktion wird die Reaktionsmischung in 100 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Hexan/Essigsäureethylester (20:1) gereinigt.
Es werden 1,24 g (56 % der Theorie) 2-Fluor-5-nitro-1-(3-thienyl)-benzol mit einem Schmelzpunkt von 65 °C erhalten.

### B. Synthese von 2-N-substituierten 2-Amino-5-amino-1-(3-thienyl)-benzolen

0,56 g (0,0025 mol) 2-Fluor-5-nitro-1-(3-thienyl)-benzol aus Stufe **A** und 5 ml des entsprechenden Amins werden in 5 ml Ethanol gelöst. Anschließend wird die Reaktionmischung auf 80 °C erwärmt. Nach Beendigung der Reaktion wird die Reaktionmischung in 50 g Eis gegossen, mit Essigsäureethylester extrahiert und sodann mit Magnesiumsulfat getrocknet. Das Lösungsmittel wird am Rotationsverdampfer abdestilliert und der Rückstand an Kieselgel mit Dichlormethan/Ethanol (50:1) gereinigt. Das so erhaltene Produkt wird in 30 ml Ethanol gelöst und unter Zusatz von 100 mg eines Palladium-Aktivkohle-Katalysators (10 %ig) bei 50 °Celsius hydriert. Nach Aufnahme der erforderlichen Wasserstoff-menge wird vom Katalysator abfiltriert und das Lösungsmittel am Rotationsverdampfer abdestilliert. Anschließend werden zur Herstellung des Hydrochlorides 5 ml einer 2,9 molaren ethanolischen Salzsäurelösung zugetropft. Der Niederschlag wird abfiltriert, zweimal mit 10 ml Ethanol gewaschen und sodann getrocknet.

### a. 2-Dimethylamino-5-amino-1-(3-thienyl)-benzol-dihydrochlorid

Verwendetes Amin: Dimethylamin
Ausbeute: 0,27 g (36 % der Theorie)
Schmelzpunkt: 232 °Celsius (Zersetzung) (farblose Kristalle)

| CHN-Analyse: | | | |
|---|---|---|---|
| (C₁₂H₁₆N₂Cl₂S) | % C | % H | % N |
| berechnet | 49,48 | 5,54 | 9,61 |
| gefunden | 48,77 | 5,84 | 9,46 |

### b. 2-Pyrrolidino-5-amino-1-(3-thienyl)-benzol-dihydrochlorid

Verwendetes Amin: Pyrrolidin
Ausbeute: 0,55 g (69 % der Theorie)
Schmelzpunkt: 205 °Celsius (Zersetzung) (farblose Kristalle)

| CHN-Analyse: | | | |
|---|---|---|---|
| (C₁₄H₁₈N₂Cl₂S) | %C | %H | % N |
| berechnet | 52,99 | 5,72 | 8,83 |
| gefunden | 52,00 | 5,77 | 8,60 |

### c. 2-Di(2-Hydroxyethyl)amino-5-amino-1-(3-thienyl)-benzol-dihydrochlorid

Verwendetes Amin: Diethanolamin
Ausbeute: 0,14 g (16 % der Theorie)
Schmelzpunkt: 208 °Celsius (Zersetzung) (farblose Kristalle)

| CHN-Analyse: | | | |
|---|---|---|---|
| (C₁₄H₂₀N₂O₂Cl₂S) | % C | % H | % N |
| berechnet | 47,86 | 5,74 | 7,97 |
| gefunden | 47,33 | 5,74 | 7,90 |

### d. 2-(2-Hydroxyethyl)amino-5-amino-1-(3-thienyl)-benzol-dihydrochlorid

Verwendetes Amin: Ethanolamin
Ausbeute: 0,5 g (42 % der Theorie)
Schmelzpunkt: 208 °Celsius (Zersetzung) (farblose Kristalle)

| CHN-Analyse: | | | |
|---|---|---|---|
| (C₁₂H₁₆N₂OCl₂S) | % C | % H | % N |
| berechnet | 46,91 | 5,25 | 9,12 |
| gefunden | 46,45 | 5,43 | 9,13 |

### e. 2-(2-Methoxyethyl)amino-5-amino-1-(3-thienyl)-benzol-dihydrochlorid

Verwendetes Amin 2-Methoxyethylamin
Ausbeute: 0,50 g (42 % der Theorie)
Schmelzpunkt: 208 °Celsius (Zersetzung) (farblose Kristalle)

| CHN-Analyse: | | | |
|---|---|---|---|
| (C₁₃H₁₈Cl₂N₂OS) | % C | % H | % N |
| berechnet | 48,60 | 5,65 | 8,72 |
| gefunden | 48,24 | 6,43 | 8,38 |

### f. 2-(2,3-Dihydroxypropyl)amino-5-amino-1-(3-thienyl)-benzol-dihydrochlorid

Verwendetes Amin 2,3-Dihydroxypropylamin
Ausbeute: 0,35 g (30 % der Theorie)
Schmelzpunkt: 208 °Celsius (Zersetzung) (farblose Kristalle)

| CHN-Analyse: | | | |
|---|---|---|---|
| (C₁₃H₁₈Cl₂N₂OS) | % C | % H | % N |
| berechnet | 46,3 | 5,38 | 8,31 |
| gefunden | 44,08 | 6,07 | 8,00 |

### Beispiel 3: Synthese von 2,5-Diamino-4-methyl-1-(3-thienyl)-benzol

1,87 g (0,01 mol) 5-Chlor-2-methyl-4-nitroanilin und 0,013 mol Thiophen-3-borsäure werden unter Argon in 70 ml 1,2-Dimethoxyethan gelöst. Anschließend werden 0,5 g Tetrakis-(triphenylphosphin)-palladium (0,0005 mol) und 13 ml 2N Kaliumcarbonat-lösung zugegeben und die Reaktionsmischung auf 80 °C erwärmt. Nach Beendigung der Reaktion wird die Reaktionsmischung in 100 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Hexan/Essigsäureethylester (20:1) gereinigt.
Das so erhaltene Produkt wird in 30 ml Ethanol gelöst und unter Zusatz von 100 mg eines Palladium-Aktivkohle-Katalysators (10 %ig) bei 50 °C hydriert. Nach Aufnahme der erforderlichen Wasserstoff-menge wird vom Katalysator abfiltriert und das Lösungsmittel am Rotationsverdampfer abdestilliert.
Anschließend werden zur Herstellung des Hydrochlorides 5 ml einer 2,9 molaren ethanolischen Salzsäurelösung zugetropft. Der Niederschlag wird abfiltriert, zweimal mit 10 ml Ethanol gewaschen und sodann getrocknet. Ausbeute: 2,1 g (76 % der Theorie)

| CHN-Analyse: | | | |
|---|---|---|---|
| (C₁₁H₁₄Cl₂N₂S) | % C | % H | % N |
| berechnet | 47,66 | 5,09 | 10,11 |
| gefunden | 48,18 | 5,57 | 9,62 |

### Beispiel 4: Synthese von 2,5-Diamino-4-methoxy-1-(3-thienyl)-benzol

1,87 g (0,01 mol) 5-Chlor-2-methoxy-4-nitroanilin und 0,013 mol Thiophen-3-borsäure werden unter Argon in 70 ml 1,2-Dimethoxyethan gelöst. Anschließend werden 0,5 g Tetrakis-(triphenylphosphin)-palladium (0,0005 mol) und 13 ml 2N Kaliumcarbonat-lösung zugegeben und die Reaktionsmischung auf 80 °C erwärmt. Nach Beendigung der Reaktion wird die Reaktionsmischung in 100 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Hexan/Essigsäureethylester (20:1) gereinigt.
Das so erhaltene Produkt wird in 30 ml Ethanol gelöst und unter Zusatz von 100 mg eines Palladium-Aktivkohle-Katalysators (10 %ig) bei 50 °C hydriert. Nach Aufnahme der erforderlichen Wasserstoff-menge wird vom Katalysator abfiltriert und das Lösungsmittel am Rotationsverdampfer abdestilliert.
Anschließend werden zur Herstellung des Hydrochlorides 5 ml einer 2,9 molaren ethanolischen Salzsäurelösung zugetropft Der Niederschlag wird abfiltriert, zweimal mit 10 ml Ethanol gewaschen und sodann getrocknet. Ausbeute: 1,8 g (62 % der Theorie)

| CHN-Analyse: | | | |
|---|---|---|---|
| (C₁₁H₁₄Cl₂N₂OS) | % C | % H | % N |
| berechnet | 45,06 | 4,81 | 9,55 |
| gefunden | 44,98 | 4,86 | 9,50 |

### Beispiele 5 bis 13: Haarfärbemittel

Es werden Haarfärbelösungen der folgenden Zusammensetzunq hergestellt:

| | |
|---|---|
| 0,0125 mol | Entwicklersubstanz gemäß Tabelle 1 |
| 0,0125 mol | Kupplersubstanz gemäß Tabelle 1 |
| 10,0 g | Kaliumoleat (8prozentige wäßrige Lösung) |
| 10,0 g | Ammoniak (22prozentige wäßrige Lösung) |
| 10,0 g | Isopropanol |
| 0,3 g | Ascorbinsäure |
| ad 100,0 g | Wasser |

30 g der oben beschriebenen Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40° C wird das Haar mit Wasser gespült, mit einem Shampoo gewaschen und getrocknet. Die resultierenden Färbungen sind in der nachfolgenden Tabelle 1 zusammengefaßt.

**Tabelle 1**

| **Beispiel** | **Entwicklersubstanz der Formel (I)** | **Kupplersubstanz** | **erhaltene Färbung** |
|---|---|---|---|
| **6** | 2,5-Diamino-1-(3-thienyl)-benzol*2HCl | 2-Amino-4-(2-hydroxyethyl)-amino-anisolsulfat | dunkelblau |
| **7** | 2,5-Diamino-1-(3-thienyl)-benzol*2HCl | 1,3-Dihydroxybenzol | dunkelblond |
| **8** | 2,5-Diamino-1-(3-thienyl)benzol*2HCl | 1-Chlor-2,4-dihydroxy-benzol | dunkelblond |
| **9** | 2-Di(2-Hydroxyethyl)-amino-5-amino-1-(3-thienyl)benzol*2HCl | 1,3-Dihydroxybenzol | blond |
| **10** | 2-Pyrrolidino-5-amino-1-(3-thienyl)benzol *2HCl | 2-Amino-4-(2-hydroxyethyl)-amino-anisolsulfat | blau |
| **11** | 2-(2-Hydroxyethyl)amino-5-amino-1-(3-thienyl)-benzol*2HCl | 1,3-Dihydroxybenzol | blond |
| **12** | 2-(2-Methoxyethyl)amino-5-amino-1-(3-thienyl) benzol*2HCl | 2-Amino-4-(2-hydroxyethyl)-amino-anisolsulfat | blau |
| **13** | 2-(2,3-Dihydroxypropyl)-amino-5-amino-1-(3-thienyl)-benzol*2HCl | 1,3-Dihydroxybenzol | blond |

### Beispiele 14 bis 21: Haarfärbemittel

Es werden Haarfärbelösungen der folgenden Zusammensetzung hergestellt:

| | |
|---|---|
| 0,0000125 mol | Entwicklersubstanz der Formel (I) gemäß Tabelle 2 |
| 0,0000125 mol | Kupplersubsianz gemäß Tabelle 2 |
| 0,01g | Kaliumoleat (8prozentige wäßrige Lösung) |
| 0,01g | Ammoniak (22prozentige wäßrige Lösung) |
| 0,01g | Ethanol |
| 0,003 g | Ascorbinsäure |
| ad 1,0 g | Wasser |

1 g der vorstehenden Färbelösung werden unmittelbar vor der Anwendung mit 1 g einer 6prozentigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 2 zusammengefaßt.

**Tabelle 2:**

| **Beispiel** | **Entwicklersubstanz der Formel (I)** | **Kupplersubstanz** | **erhaltene Färbung** |
|---|---|---|---|
| **14** | 2,5-Diamino-1-(5-methyl-3-thienyl)benzol *2HCl | 1,3-Diamino-4-(2-hydroxyethoxy)-benzolamino-anisolsulfat | dunkelblau |
| **15** | 2,5-Diamino-1-(5-methyl-3-thienyl)benzol *2HCl | **Resorcin** | dunkelblond |
| **16** | 2,5-Diamino-1-(2-chlor-3-thienyl)benzol*2HCl | **Resorcin** | dunkelblond |
| **17** | 2,5-Diamino-1-(3-furyl)benzol *2HCl | 1,3-Diamino-4-(2-hydroxyethoxy)-benzolamino-anisolsulfat | dunkelblau |
| **18** | 2,5-Diamino-1-(3-furyl)benzol *2HCl | Resorcin | dunkelblond |
| **19** | 2,5-Diamino-1-(4-(2-hydroxethyl)aminomethyl-3-thienyl)-benzol *2HCl | 1,3-Diamino-4-(2-hydroxyethoxy)-benzolamino-anisolsulfat | blau |
| **20** | 2,5-Diamino-1-(4-propylaminomethyl-3-thienyl)-benzol *2HCl | Resorcin | blond |
| **21** | 2,5-Diamino-1-(4-(phenyl)aminomethyl-3-thienyl)-benzol *2HCl | Resorcin | blond |

### Beispiel 22: Haarfärbemittel

Es werden Haarfärbelösungen der folgenden Zusammensetzung hergestellt:

| | |
|---|---|
| 0,160 g | 2,5-Diamino-1-(3-thienyl)benzol*2HCl |
| 0,160 g | 1,4-Diamino-2-(2-hydroxyethyl)benzol*sulfat |
| 0,137 g | 1,3-Dihydroxy-Benzol |
| 0,100 g | 1,3-Dihydroxy-2-methylbenzol |
| 0,100 g | 2-Amino-5-methyl-phenol |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Das Haar hat eine blonde Färbung erhalten.

### Beispiel 23: Haarfärbemittel

Es werden Haarfärbelösungen der folgenden Zusammensetzung hergestellt:

| | |
|---|---|
| 0,320 g | 2,5-Diamino-1-(3-thienyl)benzol*2HCl |
| 0,300 g | 5-Amino-2-methylphenol |
| 0,600 g | 4-Amino-3-methylphenol |
| 0,600 g | 4-Amino-phenol |
| 0,100 g | α-Naphtol |
| 0,200 g | 2-Chlor-6-(ethylamino)-4-nitrophenol |
| 10,000 g | Kaliumoleat (8prozentige wäßrige Lösung) |
| 10,000 g | Ammoniak (22prozentige wäßrige Lösung) |
| 10,0,00 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Das Haar hat eine rote Färbung erhalten.

### Beispiel 24: Haarfärbemittel

Es werden Haarfärbelösungen der folgenden Zusammensetzung hergestellt:

| | |
|---|---|
| 0,320 g | 2,5-Diamino-1-(3-thienyl)benzol*2HCl |
| 0,040 g | 5-Amino-2-methylphenol |
| 0,090 g | 2-Amino-4-(2'-hydroxyethyl)-amino-anisolsulfat |
| 0,030 g | 3-Aminophenol |
| 0,030 g | 1,3-Dihydroxy-benzol |
| 0,040 g | 1,3-Dihydroxy-2-methylbenzol |
| 0,100 g | 4-Amino-5-methylphenol |
| 0,200 g | 2-Amino-3-methylphenol |
| 0,100 g | 2-Amino-6-methylphenol-hydrochlorid |
| 0,010 g | 4-Chlor-N-(2-hydroxyethyl)-2-nitroanilin |
| 0,020 g | 2-Amino-4,6-dinitrophenol |
| 0,100 g | 2-Chlor-6-(ethylamino)-4-nitrophenol |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Das Haar hat eine braune Färbung erhalten.

### Beispiel 25: Haarfärbemittel

Es werden Haarfärbelösungen der folgenden Zusammensetzung hergestellt:

| | |
|---|---|
| 0,320 g | 2,5-Diamino-1-(3-thienyl)benzol*2HCl |
| 0,040 g | 5-Amino-2-methylphenol |
| 0,050 g | 1,3-Diamino-4-(2-hydroxyethoxy)-benzol |
| 0,030 g | 3-Aminophenol |
| 0,030 g | 1,3-Dihydroxy-benzol |
| 0,040 g | 1,3-Dihydroxy-2-methylbenzol |
| 0,100 g | 4-Amino-5-methylphenol |
| 0,200 g | 2-Amino-3-methylphenol |
| 0,100 g | 2-Amino-6-methylphenol-hydrochlorid |
| 0,010 g | 4-Chlor-N-(2-hydroxyethyl)-2-nitroanilin |
| 0,020 g | 2-Amino-4,6-dinitrophenol |
| 0,100 g | 2-Chlor-6-(ethylamino)-4-nitrophenol |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Das Haar hat eine braune Färbung erhalten.

### Beispiel 26: Haarfärbemittel

Es werden Haarfärbelösungen der folgenden Zusammensetzung hergestellt:

| | |
|---|---|
| 0,220 g | 2,5-Diamino-1-(3-thienyl)benzol*2HCl |
| 0,100 g | 1,4-Diamino-2-(2-hydroxyethyl)benzol*sulfat |
| 0,020 g | 5-Amino-2-methylphenol |
| 0,010 g | 1,3-Diamino-4-(2-hydroxyethoxy)-benzol |
| 0,004 g | 2-Amino-4-(2'-hydroxyethyl)-amino-anisolsulfat |
| 0,020 g | 1,3-Dihydroxy-benzol |
| 0,040 g | 1,3-Dihydroxy-2-methylbenzol |
| 0,008 g | 4-Amino-3-methylphenol |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Das Haar hat eine braune Färbung erhalten.

### Beispiel 27: Haarfärbemittel

Es werden Haarfärbelösungen der folgenden Zusammensetzung hergestellt:

| | |
|---|---|
| 0,220 g | 2,5-Diamino-1-(3-thienyl)benzol*2HCl |
| 0,100 g | 4-Di-(2-hydroxyethyl)amino-anilin-sulfat |
| 0,020 g | 5-Amino-2-methylphenol |
| 0,010 g | 1,3-Diamino-4-(2-hydroxyethoxy)-benzol |
| 0,015 g | 2-Amino-4-(2'-hydroxyethyl)-amino-anisclsulfat |
| 0,020 g | 1,3-Dihydroxy-benzol |
| 0,040 g | 1,3-Dihydroxy-2-methylbenzol |
| 0,008 g | 4,5-Diamino-1-(2-hydroxyethyl)-1H-pyrazol-sulfat |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Das Haar hat eine braune Färbung erhalten.

### Beispiel 28: Haarfärbemittel

Es werden Haarfärbelösungen der folgenden Zusammensetzung hergestellt:

| | |
|---|---|
| 0,320 g | 2,5-Diamino-1-(3-thienyl)benzol*2HCl |
| 0,020 g | 5-Amino-2-methylphenol |
| 0,010 g | 1,3-Diamino-4-(2-hydroxyethoxy)-benzol |
| 0,015 g | 2-Amino-4-(2'-hydroxyethyl)-amino-anisolsulfat |
| 0,020 g | 1,3-Dihydroxy-benzol |
| 0,040 g | 1,3-Dihydroxy-2-methylbenzol |
| 0,008 g | 4-Amino-2-(aminomethyl)phenol-dihydrochlorid |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Das Haar hat eine braune Färbung erhalten.

Alle Gewichtsangaben stellen, soweit nicht anders angegeben, Gewichteprozente dar.

## Patentansprüche

1. p-Diaminobenzol-Derivate der allgemeinen Formel (I) 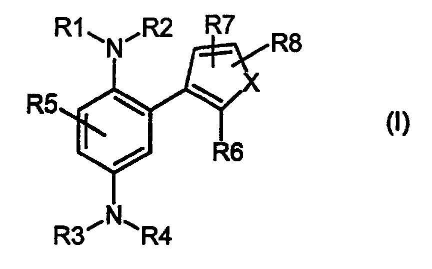 worin
**X** gleich Sauerstoff, Schwefel, Selen oder N-R9 ist;
**R1,R2,R3 und R4** unabhängig voneinander Wasserstoff, eine C₁-C₆-Alkylgruppe, eine C₁-C₄-Hydroxyalkylgruppe, eine C₂-C₄ Dihydroxyalkylgruppe oder eine C₁-C₄-Alkoxy-(C₁-C₂)-alkylgruppe darstellen oder R1 und R2 beziehungsweise R3 und R4 einen viergliedrigen bis achtgliedrigen aliphatischen Ring bilden, wobei mindestens 2 der Reste R1 bis R4 Wasserstoff darstellen;
**R5** gleich Wasserstoff, einem Halogenatom, einer C₁-C₄-Alkylgruppe, einer C₁-C₄ -Hydroxyalkylgruppe oder einer C₁-C₄-Alkoxygruppe ist;
**R6** gleich Wasserstoff, einer Hydroxygruppe, einem Halogenatom, einer Cyanogruppe, einer C₁-C₄-Alkoxygruppe, einer C₁-C₆ -Alkylgruppe, einer C₁-C₄-Alkylthioethergruppe, einer Mercaptogruppe, einer Nitrogruppe, einer Aminogruppe, einer Alkylaminogruppe, einer Dialkylaminogruppe, einer -C(O)H-Gruppe, einer -C(O)CH₃-Gruppe, einer -C(O)CF₃-Gruppe, einer -Si(CH₃)₃-Gruppe, einer C₁-C₄-Hydroxyalkylgruppe, einer C₃-C₄-Dihydroxyalkylgruppe, einer -CH=CHR10-Gruppe, einer -(CH₂)ₚ-CO₂R11-Gruppe oder einer -(CH₂)ₚ-R12-Gruppe mit p=1,2,3 oder 4, einer -C(R13)=NR14-Gruppe oder einer C(R16)H-NR17R18-Gruppe ist;
**R7 und R8** unabhängig voneinander gleich Wasserstoff, einem Halogenatom, einer Cyanogruppe, einer Hydroxygruppe, einer C₁-C₄-Alkoxygruppe, einer C₁-C₆ Alkylgruppe, einer C₁-C₄-Alkylthioethergruppe, einer Mercaptogruppe, einer Nitrogruppe, einer Aminogruppe, einer Alkylamino-gruppe, einer Dialkylaminogruppe, einer -C(O)H-Gruppe, einer -C(O)CH₃-Gruppe, einer -C(O)CF₃-Gruppe, einer -Si(CH₃)₃-Gruppe, einer C₁-C₄-Hydroxyalkylgruppe, einer C₃-C₄-Dihydroxyalkylgruppe, einer -CH=CHR10-Gruppe, einer -(CH₂)ₚ-CO₂R11-Gruppe oder einer -(CH₂)ₚ-R12-Gruppe mit p=1,2,3 oder 4, einer -C(R13)=NR14-Gruppe oder einer C(R16)H-NR17R18-Gruppe sind;
**R9** gleich Wasserstoff, einer C₁-C₆-Alkylgruppe, einer C₁-C₄-Hydroxyalkylgruppe, einer Phenylgruppe oder einer Acetylgruppe ist;
**R10** gleich Wasserstoff, einer Hydroxygruppe, einer Nitrogruppe, einer Aminogruppe, einer -CO₂R11-Gruppe oder einer -C(O)CH₃-Gruppe ist;
**R11, R13 und R16** unabhänging voneinander gleich Wasserstoff oder einer C₁-C₄-Alkylgruppe sind;
**R12** gleich einer Aminogruppe oder einer Nitrilgruppe ist;
**R14, R17 und R18** unabhängig voneinander gleich Wasserstoff, einer Hydroxygruppe, einer C₁-C₄-Alkylgruppe, einer C₁-C₄-Hydroxyalkylgruppe, einer C₃-C₄-Dihydroxyalkylgruppe oder einem Rest der Formel (II) sind 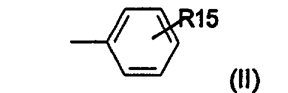 und
**R15** gleich Wasserstoff, einer Aminogruppe oder einer Hydroxygruppe ist, oder deren physiologisch verträgliche, wasserlösliche Salze.

2. p-Diaminobenzol-Derivat nach Anspruch 1, **dadurch gekennzeichnet, daß** in der Formel (I) einer oder mehrere der Reste R5 bis R8 gleich Wasserstoff sind.

3. p-Diaminobenzol-Derivat nach Anspruch 1 oder 2, **dadurch gekennzeichnet, daß** in der Formel (I) die Reste R1, R2, R3 und R4 gleich Wasserstoff sind.

4. p-Diaminobenzol-Derivat nach einem der Ansprüche 1 bis 3, **dadurch gekennzeichnet, daß** in der Formel (I) R7 gleich Wasserstoff und R6 gleich Wasserstoff, -C(O)H, -C(O)CH₃, C₁-C₄-Alkyl oder C₁-C₄-Hydroxyalkyl ist.

5. p-Diaminobenzol-Derivate nach Anspruch 4, **dadurch gekennzeichnet, daß** in der Formel (I) die Reste R7 und R6 gleich Wasserstoff sind.

6. p-Diaminobenzol-Derivat nach einem der Ansprüche 1 bis 5, **dadurch gekennzeichnet, daß** es ausgewählt ist aus 2,5-Diamino-1-(5-chlor-3-thienyl)benzol; 2,5-Diamino-1-(3-furyl)benzol; 2,5-Diamino-1-(2-acetyl-3-thienyl)benzol; 2,5-Diamino-1-(pyrrol-3-yl)benzol; 2,5-Diamino-1-(2-formyl-3-thienyl)benzol; 2,5-Diamino-1-(4-formyl-3-thienyl)-benzol; 2,5-Diamino-1-(2-formyl-3-furyl)benzol; 2,5-Diamino-1-(4-formyl-3-furyl)benzol; 2,5-Diamino-1-(5-methyl-3-thienyl)benzol, 2,5-Diamino-1-(5-methyl-3-thienyl)benzol, 2,5-Diamino-1-(4-(4-nitropropenyl)-3-thienyl)benzol, 2,5-Diamino-1-(3-furyl)benzol, 2,5-Diamino-1-(4-formyl-3-thienyl)benzol, 2,5-Diamino-1-(4-(2-hydroxyethyl)aminomethyl-3-thienyl)-benzol, 2,5-Diamino-1-(4-pyrrolidinomethyl-3-thienyl)-benzol, 2,5-Diamino-1-(4-(dimethylaminoethyl)aminomethyl-3-thienyl)-benzol, 2,5-Diamino-1-(4-(2-methoxy-5-chlor-phenyl)aminomethyl-3-thienyl)-benzol, 2,5-Diamino-1-(4-(3-ethoxypropyl)aminomethyl-3-thienyl)-benzol, 2,5-Diamino-1-(4-(methoxyethyl)aminomethyl-3-thienyl)-benzol, 2,5-Diamino-1-(4-(3,4-dimethoxy-phenyl)aminomethyl-3-thienyl)benzol, 2,5-Diamino-1-(4-(4-trifluormethyl-benzyl)aminomethyl-3-thienyl)benzol, 2,5-Diamino-1-(4-(3-methoxy-phenyl)aminomethyl-3-thienyl)-benzol, 2,5-Diamino-1-(4-(O,N-dimethyl-hydroxylaminomethyl-3-thienyl)-benzol, 2,5-Diamino-1-(4-(2,4-dimethoxy-5-chlor-phenyl)aminomethyl-3-thienyl)-benzol, 2,5-Diamino-1-(4-(4-phenoxy-phenyl)aminomethyl-3-thienyl)-benzol, 2,5-Diamino-1-(4-(2-diphenyl)aminomethyl-3-thienyl)-benzol, 2,5-Diamino-1-(4-(3,4-dimethoxybenzyl)aminomethyl-3-thienyl)-benzol, 2,5-Diamino-1-(4-(4-methoxyphenyl)aminomethyl-3-thienyl)-benzol, 2,5-Diamino-1-(4-(4-hydroxy-butyl)aminomethyl-3-thienyl)-benzol, 2,5-Diamino-1-(4-(2-methoxy-3-fluor-phenyl)aminomethyl-3-thienyl)-benzol, 2,5-Diamino-1-(4-(4-(1-phenyl-ethyl)aminomethyl-3-thienyl)-benzol, 2,5-Diamino-1-(4-(2-furyl)aminomethyl-3-thienyl)-benzol, 2,5-Diamino-1-(4-(2-pyridyl)aminomethyl-3-thienyl)-benzol, 2,5-Diamino-1-(4-morpholinomethyl-3-thienyl)-benzol, 2,5-Diamino-1-(4-4-benzyl-pyperazinomethyl-3-thienyl)-benzol, 2,5-Diamino-1-(4-(3-carboxamid-phenyl)aminomethyl-3-thienyl)-benzol, 2,5-Diamino-1-(4-(N-methyl-N-phenyl)aminomethyl-3-thienyl)-benzol, 2,5-Diamino-1-(4-diethylaminomethyl-3-thienyl)-benzol, 2,5-Diamino-1-(4-(pyridin-4yl-methyl)aminomethyl-3-thienyl)-benzol, 2,5-Diamino-1-(4-(3,5-dimethoxy-benzyl)aminomethyl-3-thienyl)-benzol, 2,5-Diamino-1-(4-(4-morpholino-phenyl)aminomethyl-3-thienyl)-benzol, 2,5-Diamino-1-(4-(1-(2-hydroxyethyl))propylaminomethyl-3-thienyl)-benzol, 2,5-Diamino-1-(4-cyclopropylaminomethyl-3-thienyl)-benzol, N-(2-{[4-(2,5-Diamino-phenyl)-thiophen-2-ylmethyl]-amino}-ethyl)-acetamid, 2,5-Diamino-1-(4-cyclohexylaminomethyl-3-thienyl)-benzol, 2,5-Diamino-1-(4-propylaminomethyl-3-thienyl)-benzol, 2,5-Diamino-1-(4-(o-tolyl)aminomethyl-3-thienyl)-benzol, 2,5-Diamino-1-(4-(N-methyl-N-cyclohexyl)aminomethyl-3-thienyl)-benzol, 2,5-Diamino-1-(4-(2,6-dimethyl-morpholino)methyl-3-thienyl)-benzol, 2,5-Diamino-1-(4-(3,5-dimethyl-piperidino)methyl-3-thienyl)-benzol, 2,5-Diamino-1-(4-(3-(1-hydroxyethyl)phenyl)aminomethyl-3-thienyl)-benzol, 2,5-Diamino-1-(4-(3,4-dimethyl-phenyl)aminomethyl-3-thienyl)-benzol, 2,5-Diamino-1-(4-(3-methylmercapto-phenyl)aminomethyl-3-thienyl)-benzol, 2,5-Diamino-1-(4-(4-diphenyl)aminomethyl-3-thienyl)-benzol, 2,5-Diamino-1-(4-(4-isopropyl-phenyl)aminomethyl-3-thienyl)-benzol, 2,5-Diamino-1-(4-pentylaminomethyl-3-thienyl)benzol, 2,5-Diamino-1-(4-(dibutyl)aminomethyl-3-thienyl)-benzol, 2,5-Diamino-1-(4-isopropylaminomethyl-3-thienyl)-benzol, 2,5-Diamino-1-(4-(N-Cyclopropylmethyl-N-propyl)aminomethyl-3-thienyl)-benzol, 2,5-Diamino-1-(4-(4-hydroxy)piperidinomethyl-3-thienyl)-benzol, 2,5-Diamino-1-(4-(fluorenyl)aminomethyl-3-thienyl)-benzol, 2,5-Diamino-1-(4-(4-Dimethylamino-phenyl)aminomethyl-3-thienyl)-benzol, 2,5-Diamino-1-(4-(3,5-dlmethyl-phenyl)aminomethyl-3-thienyl)-benzol, 2,5-Diamino-1-(4-(4-methoxy-benzyl)aminomethyl-3-thienyl)-benzol, 2,5-Diamino-1-(4-(indanyl)aminomethyl-3-thienyl)-benzol, 2,5-Diamino-1-(4-(3-fluor-benzyl)aminomethyl-3-thienyl)-benzol, 2,5-Diamino-1-(4-(benzyl)aminomethyl-3-thienyl)-benzol, 2,5-Diamino-1-(4-(4-methyl-piperazino)aminomethyl-3-thienyl)-benzol, 2,5-Diamino-1-(4-(4-tert-butyl-phenyl)aminomethyl-3-thienyl)-benzol, 2,5-Diamino-1-(4-(2-chlor-benzyl)aminomethyl-3-thienyl)-benzol, 2,5-Diamino-1-(4-(napht-1-yl-methyl)aminomethyl-3-thienyl)-benzol, 2,5-Diamino-1-(4-(phenyl)aminomethyl-3-thienyl)-benzol, 2,5-Diamino-1-(4-(4-chlor-phenyl)aminomethyl-3-thienyl)-benzol, 4-{[4-(2,5-Diamino-phenyl)-thiophen-2-ylmethyl]-amino}-benzosäuremethyl-ester und 2,5-Diamino-1-(3-thienyl)benzol.

7. Mittel zum oxidativen Färben von Keratinfasem, auf der Basis einer Entwicklersubstanz-Kupplersubstanz-Kombination, **dadurch gekennzeichnet, daß** es als Entwicklersubstanz mindestens ein Diaminobenzol-Derivat der Formel (I) nach einem der Ansprüche 1 bis 6 enthält.

8. Mittel nach Anspruch 7, **dadurch gekennzeichnet, daß** das Diaminobenzol-Derivat der Formel (I) in einer Menge von 0,005 bis 20,0 Gewichtsprozent enthält.

9. Mittel nach Anspruch 7 oder 8, **dadurch gekennzeichnet, daß** die Kupplersubstanz ausgewählt ist aus 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diaminobenzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxynaphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion.

10. Mittel einem der Ansprüche 7 bis 9, **dadurch gekennzeichnet, daß** es außer dem 1,4-Diaminobenzol-Derivat der Formel (I) zusätzlich mindestens eine weitere Entwicklersubstanz, welche ausgewählt ist aus 1,4-Diaminobenzol, 2,5-Diaminotoluol, 2,5-Diaminophenylethylalkohol, 4-Aminophenol und seinen Derivaten, 4,5-Diaminopyrazolderivaten und Tetraaminopyrimidinen, enthält.

11. Mittel nach einem der Ansprüche 7 bis 10, **dadurch gekennzeichnet, daß** die Entwicklersubstanzen und Kupplersubstanzen, bezogen auf die Gesamtmenge des Oxidationsfärbemittel jeweils in einer Gesamtmenge von 0,005 bis 20 Gewichtsprozent enthalten sind.,

12. Mittel nach einem der Ansprüche 7 bis 11, **dadurch gekennzeichnet, daß** es zusätzlich mindestens einen direktziehenden Farbstoff enthält.

13. Mittel nach einem der Ansprüche 7 bis 12, **dadurch gekennzeichnet, daß** es einen pH-Wert von 6,8 bis 11,5 aufweist.

14. Mittel nach einem der Ansprüche 7 bis 13, **dadurch gekennzeichnet, daß** es in Form einer wäßrigen oder wäßrigalkoholischen Lösung, einer Creme, eines Gels oder einer Emulsion vorliegt

15. Mittel nach einem der Ansprüche 7 bis 14, **dadurch gekennzeichnet, daß** es ein Haarfärbemittel ist.

## Claims

1. p-Diaminobenzene derivatives of the general formula (I) : 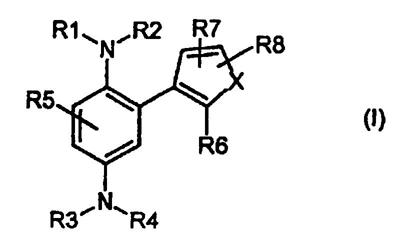
wherein X represents oxygen, sulphur, selenium or N-R⁹; R¹, R², R³ and R⁴ each, independently of each other, represent hydrogen, a C₁- to C₆-alkyl group, a C₁- to C₄-hydroxyalkyl group, a C₂- to C₄-dihydroxyalkyl group or a C₁- to C₄-alkoxy-(C₁- to C₂)-alkyl group or R¹ and R² or R³ and R⁴ represent a four-membered to eight-membered aliphatic ring, with the proviso that at least 2 of the R¹ to R⁴ groups represent hydrogen;
R⁵ represents hydrogen, a halogen atom, a C₁- to C₄-alkyl group, a C₁- to C₄-hydroxyalkyl group or a C₁- to C₄-alkoxy group;
R⁶ represents hydrogen, a hydroxy group, a halogen atom, a cyano group, a C₁- to C₄-alkoxy group, a C₁- to C₆-alkyl group, a C₁- to C₄-alkylthioether group, a mercapto group, a nitro group, an amino group, an alkylamino group, a dialkylamino group, a -C(O)H group, a -C(O)CH₃ group, a -C(O)CF₃ group, an -Si(CH₃)₃ group, a C₁- to C₄-hydroxyalkyl group, a C₃- to C₄-dihydroxyalkyl group, a -CH=CHR¹⁰ group, a -(CH₂)ₚ-CO₂R¹¹ group or a -(CH₂)ₚ R¹² group with p=1, 2, 3 or 4, a -C(R¹³)=NR¹⁴ group or a C(R¹⁶)H-NR¹⁷R¹⁸ group;
R⁷ and R⁸ each, independently of each other, represent hydrogen, a halogen atom, a cyano group, a hydroxy group, a C₁- to C₄-alkoxy group, a C₁- to C₆-alkyl group, a C₁- to C₄-alkylthioether group, a mercapto group, a nitro group, an amino group, an alkylamino group, a dialkylamino group, a -C(O)H group, a -C(O)CH₃ group, a -C(O)CF₃ group, an -Si(CH₃)₃ group, a C₁- to C₄-hydroxyalkyl group, a C₃- to C₄-dihydroxyalkyl group, a -CH=CHR¹⁰ group, a -(CH₂)ₚ-CO₂R¹¹ group or a -(CH₂)ₚR¹² group with p=1, 2, 3 or 4, a -C(R¹³)=NR¹⁴ group or a C(R¹⁶)H-NR¹⁷R¹⁸ group;
R⁹ represents hydrogen, a C₁- to C₆-alkyl group, a C₁-to C₄-hydroxyalkyl group, a phenyl group or an acetyl group;
R¹⁰ represents hydrogen, a hydroxy group, a nitro group, an amino group, a -CO₂R¹¹ group or a -C(O)CH₃ group;
R¹¹, R¹³ and R¹⁶ each, independently of each other, represent hydrogen or a C₁- to C₄-alkyl group;
R¹² represents an amino group or a nitrile group;
R¹⁴, R¹⁷ and R¹⁸ each, independently of each other, represent hydrogen, a hydroxy group, a C₁- to C₄-alkyl group, a C₁- to C₄-hydroxyalkyl group, a C₃- to C₄-dihydroxyalkyl group or a group of formula (II): 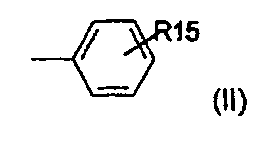 and R¹⁵ represents hydrogen, an amino group or a hydroxy group; or physiologically compatible, water-soluble salts thereof.

2. p-Diaminobenzene derivative according to Claim 1, charcterized in that one or more of said R⁵, R⁶, R⁷ and R⁸ in formula (I) represent said hydrogen.

3. p-Diaminobenzene derivative according to Claim 1 or 2, **characterized in that** each of said R¹, R², R³ and R⁴ in formula (I) represent said hydrogen.

4. p-Diaminobenzene derivative according to any of Claims 1 to 3, **characterized in that** in formula (I) said R⁷ represents said hydrogen and said R⁶ group represents said hydrogen, said -C(O)H group, said-C(O)CH₃ group, a C₁- to C₄-alkyl group, or said C₁- to C₄-hydroxyalkyl group.

5. p-Diaminobenzene derivative according to Claim 4, **characterized in that** each of said R⁷ and R⁶ in formula (I) represent said hydrogen.

6. p-Diaminobenzene derivative according to any of Claims 1 to 5, **characterized in that** it is selected from the group consisting of 2,5-diamino-1-(5-chloro-3-thienyl)benzene, 2,5-diamino-1-(3-furyl)benzene, 2,5-diamino-1-(2-acetyl-3-thienyl)benzene, 2,5-diamino-1-(pyrrol-3-yl)benzene, 2,5-diamino-1-(2-formyl-3-thienyl)benzene, 2,5-diamino-1-(4-formyl-3-thienyl)-benzene, 2,5-diamino-1-(2-formyl-3-furyl)benzene, 2,5-diamino-1-(4-formyl-3-furyl)benzene, 2,5-diamino-1-(5-methyl-3-thienyl)-benzene, 2,5-diamino-1-(5-methyl-3-thienyl)benzene, 2,5-diamino-1-(4-(4-nitropropenyl)-3-thienyl)benzene, 2,5-diamino-1-(3-furyl)benzene, 2,5-diamino-1-(4-formyl-3-thienyl)benzene, 2,5-diamino-1-(4-(2-hydroxyethyl)amino-methyl-3-thienyl)-benzene, 2,5-diamino-1-(4-pyrrolidinomethyl-3-thienyl)-benzene, 2,5-diamino-1-(4-(dimethylaminoethyl)aminomethyl-3-thienyl)benzene, 2,5-diamino-1-(4-(2-methoxy-5-chloro-phenyl)aminomethyl-3-thienyl)benzene, 2,5-diamino-1-(4-(3-ethoxypropyl)aminomethyl-3-thienyl)-benzene, 2,5-diamino-1-(4-(methoxyethyl)-aminomethyl-3-thienyl)-benzene, 2,5-diamino-1-(4-(3,4-dimethoxyphenyl)amino-methyl-3-thienyl)-benzene, 2,5-diamino-1-(4-(4-tri-fluoromethyl-benzyl)aminomethyl-3-thienyl)benzene, 2,5-diamino-1-(4-(3-methoxyphenyl)aminomethyl-3-thienyl)benzene, 2,5-diamino-1-(4-(O,N-dimethyl-hydroxylaminomethyl-3-thienyl)benzene, 2,5-diamino-1-(4-(2,4-dimethoxy-5-chlorophenyl)aminomethyl-3-thienyl)benzene, 2,5-diamino-1-(4-(4-phenoxyphenyl)-aminomethyl-3-thienyl)benzene, 2,5-diamino-1-(4-(2-diphenyl)aminomethyl-3-thienyl)benzene, 2,5-diamino-1-(4-(3,4-dimethoxybenzyl)aminomethyl-3-thienyl)benzene, 2,5-diamino-1-(4-(4-methoxyphenyl)aminomethyl-3-thienyl)benzene, 2,5-diamino-1-(4-(4-hydroxybutyl)-aminomethyl-3-thienyl)-benzene, 2,5-diamino-1-(4-(2-methoxy-3-fluorophenyl)aminomethyl-3-thienyl)benzene, 2,5-diamino-1-(4-(4-(1-phenylethyl)aminomethyl-3-thienyl)benzene, 2,5-diamino-1-(4-(2-furyl)aminomethyl-3-thienyl)benzene, 2,5-diamino-1-(4-(2-pyridyl)amino-methyl-3-thienyl)benzene, 2,5-diamino-1-(4-morpholino-methyl-3-thienyl)-benzene, 2,5-diamino-1-(4-(4-benzyl)-pyperazinomethyl-3-thienyl)benzene, 2,5-diamino-1-(4-(3-carboxamidophenyl)aminomethyl-3-thienyl)benzene, 2,5-diamino-1-(4-(N-methyl-N-phenyl)-aminomethyl-3-thienyl)benzene, 2,5-diamino-1-(4-diethyl-aminomethyl-3-thienyl)benzene, 2,5-diamino-1-(4-(pyridin-4-yl-methyl)aminomethyl-3-thienyl)benzene, 2,5-diamino-1-(4-(3,5-dimethoxybenzyl)aminomethyl-3-thienyl)benzene, 2,5-diamino-1-(4-(4-morpholinophenyl)aminomethyl-3-thienyl)-benzene, 2,5-diamino-1-(4-(1-(2-hydroxyethyl))propyl-aminomethyl-3-thienyl)benzene, 2,5-diamino-1-(4-cyclopropyl-aminomethyl-3-thienyl)benzene, N-(2-{[4-(2,5-diaminophenyl)-thiophen-2-yl-methyl]-amino}ethyl)acetamide, 2,5-diamino-1-(4-cyclohexylaminomethyl-3-thienyl)benzene, 2,5-diamino-1-(4-propylaminomethyl-3-thienyl)benzene, 2,5-diamino-1-(4-(o-tolyl)aminomethyl-3-thienyl)benzene, 2,5-diamino-1-(4-(N-methyl-N-cyclohexyl)aminomethyl-3-thienyl)benzene, 2,5-diamino-1-(4-(2,6-dimethylmorpholino)methyl-3-thienyl)benzene, 2,5-diamino-1-(4-(3,5-dimethylpiperidino)methyl-3-thienyl)benzene, 2,5-diamino-1-(4-(3-(1-hydroxyethyl)-phenyl)-aminomethyl-3-thienyl)benzene, 2,5-diamino-1-(4-(3,4-dimethyl-phenyl)aminomethyl-3-thienyl)benzene, 2,5-diamino-1-(4-(3-methylmercaptophenyl)aminomethyl-3-thienyl)benzene, 2,5-diamino-1-(4-(4-diphenyl)amino-methyl-3-thienyl)benzene, 2,5-diamino-1-(4-(4-iso-propylphenyl)aminomethyl-3-thienyl)benzene, 2,5-diamino-1-(4-pentyl-aminomethyl-3-thienyl)benzene, 2,5-diamino-1-(4-(dibutyl)aminomethyl-3-thienyl)benzene, 2,5-diamino-1-(4-isopropyl-aminomethyl-3-thienyl)benzene, 2,5-diamino-1-(4-(N-cyclopropylmethyl-N-propyl)aminomethyl-3-thienyl)benzene, 2,5-diamino-1-(4-(4-hydroxy)piperidinomethyl-3-thienyl)benzene, 2,5-diamino-1-(4-(fluorenyl)aminomethyl-3-thienyl)benzene, 2,5-diamino-1-(4-(4-dimethylaminophenyl)-aminomethyl-3-thienyl)benzene, 2,5-diamino-1-(4-(3,5-dimethylphenyl)-aminomethyl-3-thienyl)benzene, 2,5-diamino-1-(4-(4-methoxybenzyl)aminomethyl-3-thienyl)benzene, 2,5-diamino-1-(4-(indanyl)-aminomethyl-3-thienyl)benzene, 2,5-diamino-1-(4-(3-fluorobenzyl)aminomethyl-3-thienyl)-benzene, 2,5-diamino-1-(4-(benzyl)aminomethyl-3-thienyl)-benzene, 2,5-diamino-1-(4-(4-methyl-piperazino)aminomethyl-3-thienyl)benzene, 2,5-diamino-1-(4-(4-tert-butylphenyl)-aminomethyl-3-thienyl)benzene, 2,5-diamino-1-(4-(2-chlorobenzyl)aminomethyl-3-thienyl)benzene, 2,5-diamino-1-(4-(naphth-1-yl-methyl)aminomethyl-3-thienyl)benzene, 2,5-diamino-1-(4-(phenyl)aminomethyl-3-thienyl)benzene, 2,5-diamino-1-(4-(4-chlorophenyl)aminomethyl-3-thienyl)benzene, 4-{[4-(2,5-diaminophenyl)thiophen-2-yl-methyl]amino}benzoic acid methyl ester and 2,5-diamino-1-(3-thienyl)benzene.

7. Composition for oxidative dyeing of keratin fibres, based on a combination of developer and coupler substances, **characterized in that** the composition comprises as developer substance at least one diaminobenzene derivative according to any of Claims 1 to 6 of the formula (I).

8. Composition according to Claim 7, **characterized in that** said diaminobenzene derivative of the formula (I) is present in an amount of from 0.005 to 20.0 per cent by weight.

9. Composition according to Claim 7 or 8, **characterized in that** said coupler substance is selected from the group consisting of 2,6-diaminopyridine, 2-amino-4-[(2-hydroxyethyl)amino]-anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxy-ethoxy)-5-methylbenzene, 2,4-di[(2-hydroxyethyl)amino]-1,5-dimethoxy-benzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 2,4-diamino-1,5-di-(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di-(2-hydroxyethyl)-amino]aniline, 4-amino-2-di[(2-hydroxyethyl)amino]-1-ethoxybenzene, 5-methyl-2-(1-methylethyl)phenol, 3-[(2-hydroxyethyl)amino]aniline, 3-[(2-aminoethyl)amino]-aniline, 1,3-di(2,4-diaminophenoxy)propane, di(2,4-diaminophenoxy)methane, 1,3-diamino-2,4-dimethoxybenzene, 2,6-bis-(2-hydroxyethyl)aminotoluene, 4-hydroxyindole, 3-dimethylamino-phenol, 3-diethylaminophenol, 5-amino-2-methylphenol, 5-amino-4-fluoro-2-methylphenol, 5-amino-4-methoxy-2-methylphenol, 5-amino-4-ethoxy-2-methyl-phenol, 3-amino-2,4-dichlorophenol, 5-amino-2,4-dichlorophenol, 3-amino-2-methyl-phenol, 3-amino-2-chloro-6-methylphenol, 3-aminophenol, 2-[(3-hydroxyphenyl)amino]acetamide, 5-[(2-hydroxyethyl)-amino]-2-methylphenol, 3-[(2-hydroxyethyl)amino]phenol, 3-[(2-methoxyethyl)amino]-phenol, 5-amino-2-ethylphenol, 2-(4-amino-2-hydroxyphenoxy)ethanol, 5-[(3-hydroxypropyl)amino]-2-methylphenol, 3-[(2,3-dihydroxypropyl)amino)-2-methylphenol, 3-[(2-hydroxyethyl)amino]-2-methylphenol, 2-amino-3-hydroxypyridine, 5-amino-4-chloro-2-methylphenol, 1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxy-benzene, 1-chloro-2,4-dihydroxybenzene, 2-chloro-1,3-dihydroxybenzene, 1,2-dichloro-3,5-dihydroxy-4-methyl-benzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxole, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolenedione.

10. Composition according to any of Claims 7 to 9, **characterized in that**, besides the 1,4-diaminobenzene derivative of the formula (I), it additionally comprises at least one further developer substance selected from the group consisting of 1,4-diaminobenzene, 2,5-diaminotoluene, 2,5-diaminophenylethyl alcohol, 4-aminophenol, substituted 4-aminophenols, substituted 4,5-diaminopyrazoles and tetraaminopyrimidines.

11. Composition according to any of Claims 7 to 10, **characterized in that** the developer substances and coupler substances are each present in a total amount of from 0.005 to 20 per cent by weight, based on the total amount of the oxidation dye composition.

12. Composition according to any of Claims 7 to 11, **characterized in that** it additionally comprises at least one direct-dyeing dye compound.

13. Composition according to any of Claims 7 to 12, **characterized in that** it has a pH of from 6.8 to 11.5.

14. Composition according to any of Claims 7 to 13, **characterized in that** it is in the form of an aqueous or aqueous-alcoholic solution, a cream, a gel or an emulsion.

15. Composition according to any of Claims 7 to 14, **characterized in that** it is a hair-dyeing composition.

## Revendications

1. Dérivés de p-diaminobenzène de formule générale (I) 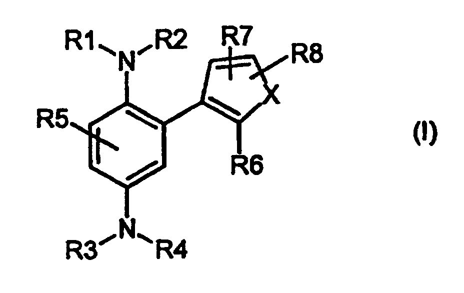 dans laquelle
**X** représente un oxygène, un soufre, un sélénium ou N-R9 ;
**R1, R2, R3 et R4** représentent, indépendamment les uns des autres, un hydrogène, un groupe alkyle en C₁-C₆, un groupe hydroxyalkyle en C₁-C₄, un groupe dihydroxyalkyle en C₂-C₄ ou un groupe C₁-C₄-alcoxy-(C₁-C₂)-alkyle, ou R1 et R2, respectivement R3 et R4 forment un noyau aliphatique à quatre à huit chaînons, au moins 2 des radicaux R1 à R4 représentant un hydrogène ;
**R5** représente un hydrogène, un atome d'halogène, un groupe alkyle en C₁-C₄, un groupe hydroxyalkyle en C₁-C₄ ou un groupe alcoxy en C₁-C₄ ;
**R6** représente un hydrogène, un groupe hydroxy, un atome d'halogène, un groupe cyano, un groupe alcoxy en C₁-C₄, un groupe alkyle en C₁-C₆, un groupe C₁-C₄-alkylthioéther, un groupe mercapto, un groupe nitro, un groupe amino, un groupe alkylamino, un groupe dialkylamino, un groupe -C(O)H, un groupe -C(O)CH₃, un groupe -C(O)CF₃, un groupe -Si(CH₃)₃, un groupe hydroxyalkyle en C₁-C₄, un groupe dihydroxyalkyle en C₃-C₄, un groupe -CH=CHR10, un groupe -(CH₂)ₚ-CO₂R11 ou un groupe -(CH₂)ₚ-R12 avec p = 1, 2, 3 ou 4, un groupe -C(R13)=NR14 ou un groupe C(R16)H-NR17R18 ;
**R7 et R8** représentent, indépendamment l'un de l'autre, un hydrogène, un atome d'halogène, un groupe cyano, un groupe hydroxy, un groupe alcoxy en C₁-C₄, un groupe alkyle en C₁-C₆, un groupe alkylthioéther en C₁-C₄, un groupe mercapto, un groupe nitro, un groupe amino, un groupe alkylamino, un groupe dialkylamino, un groupe -C(O)H, un groupe -C(O)CH₃, un groupe -C(O)CF₃, un groupe -Si(CH₃)₃, un groupe hydroxyalkyle en C₁-C₄, un groupe dihydroxyalkyle en C₃-C₄, un groupe -CH=CHR10, un groupe -(CH₂)ₚ-CO₂R11 ou un groupe -(CH₂)ₚ-R12 avec p = 1, 2, 3 ou 4, un groupe -C(R13)=NR14 ou un groupe C(R16)H-NR17R18 ;
**R9** représente un hydrogène, un groupe alkyle en C₁-C₆, un groupe hydroxyalkyle en C₁-C₄, un groupe phényle ou un groupe acétyle ;
**R10** représente un hydrogène, un groupe hydroxy, un groupe nitro, un groupe amino, un groupe -CO₂R11 ou un groupe -C(O)CH₃ ;
**R11, R13 et R16** représentent, indépendamment les uns des autres, un hydrogène ou un groupe alkyle en C₁-C₄ ;
R12 représente un groupe amino ou un groupe nitrile ;
**R14, R17 et R18** représentent, indépendamment les uns des autres, un hydrogène, un groupe hydroxy, un groupe alkyle en C₁-C₄, un groupe hydroxyalkyle en C₁-C₄, un groupe dihydroxyalkyle en C₃-C₄ ou un radical de formule (II) 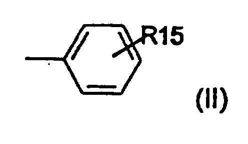 et
**R15** représente un hydrogène, un groupe amino ou un groupe hydroxy,
ou leurs sels hydrosolubles physiologiquement acceptables.

2. Dérivé de p-diaminobenzène selon la revendication 1, **caractérisé en ce que** dans la formule (I), un ou plusieurs des radicaux R5 à R8 représentent un hydrogène.

3. Dérivé de p-diaminobenzène selon la revendication 1 ou 2, **caractérisé en ce que** dans la formule (I), les radicaux R1, R2, R3 et R4 représentent un hydrogène.

4. Dérivé de p-aminobenzène selon l'une quelconque des revendications 1 à 3, **caractérisé en ce que** dans la formule (I), R7 représente un hydrogène et R6 représente un hydrogène, -C(O)H, -C(O)CH₃, un alkyle en C₁-C₄ ou un hydroxyakyle en C₁-C₄.

5. Dérivés de p-diaminobenzène selon la revendication 4, **caractérisé en ce que** dans la formule (I), les radicaux R7 et R6 représentent un hydrogène.

6. Dérivé de p-diaminobenzène selon l'une quelconque des revendications 1 à 5, **caractérisé en ce qu'**il est choisi parmi le 2,5-diamino-1-(5-chloro-3-thiényl)-benzène ; le 2,5-diamino-1-(3-furyl)benzène; le 2,5-diamino-1-(2-acétyl-3-thiényl)benzène ; le 2,5-diamino-1-(pyrrol-3-yl)benzène ; le 2,5-diamino-1-(2-formyl-3-thiényl)benzène ; le 2,5-diamino-1-(4-formyl-3-thiényl)benzène ; le 2,5-diamino-1-(2-formyl-3-furyl)-benzène ; le 2,5-diamino-1-(4-formyl-3-furyl)benzène ; le 2,5-diamino-1-(5-méthyl-3-thiényl)benzène, le 2,5-diamino-1-(5-méthyl-3-thiényl)benzène, le 2,5-diamino-1-(4-(4-nitropropényl)-3-thiényl)benzène, le 2,5-diamino-1-(3-furyl)benzène, le 2,5-diamino-1-(4-formyl-3-thiényl)benzène, le 2,5-diamino-1-(4-(2-hydroxyéthyl)aminométhyl-3-thiényl)benzène, le 2,5-diamino-1-(4-pyrrolidinométhyl-3-thiényl)benzène, le 2,5-diamino-1-(4-(diméthylaminoéthyl)aminométhyl-3-thiényl)benzène, le 2,5-diamino-1-(4-(2-méthoxy-5-chlorophényl)aminométhyl-3-thiényl)benzène, le 2,5-diamino-1-(4-(3-éthoxypropyl)aminométhyl-3-thiényl)benzène, le 2,5-diamino-1-(4-(méthoxyéthyl)aminométhyl-3-thiényl)benzène, le 2,5-diamino-1-(4-(3,4-diméthoxyphényl)aminométhyl-3-thiényl)benzène, le 2,5-diamino-1-(4-(4-trifluorométhylbenzyl)aminométhyl-3-thiényl)benzène, le 2,5-diamino-1-(4-(3-méthoxyphényl)aminométhyl-3-thiényl)-benzène, le 2,5-diamino-1-(4-(O,N-diméthylhydroxylaminométhyl-3-thiényl)benzène, le 2,5-diamino-1-(4-(2,4-diméthoxy-5-chlorophényl)aminométhyl-3-thiényl)-benzène, le 2,5-diamino-1-(4-(4-phénoxyphényl)-aminométhyl-3-thiényl)benzène, le 2,5-diamino-1-(4-(2-diphényl)aminométhyl-3-thiényl)benzène, le 2,5-diamino-1-(4-(3,4-diméthoxybenzyl)aminométhyl-3-thiényl)benzène, le 2,5-diamino-1-(4-(4-méthoxyphényl)aminométhyl-3-thiényl)benzène, le 2,5-diamino-1-(4-(4-hydroxybutyl)-aminométhyl-3-thiényl)benzène, le 2,5-diamino-1-(4-(2-méthoxy-3-fluorophényl)aminométhyl-3-thiényl)benzène, le 2,5-diamino-1-(4-(4-(1-phényléthyl)aminométhyl-3-thiényl)benzène, le 2,5-diamino-1-(4-(2-furyl)aminométhyl-3-thiényl)benzène, le 2,5-diamino-1-(4-(2-pyridyl)aminométhyl-3-thiényl)benzène, le 2,5-diamino-1-(4-morpholinométhyl-3-thiényl)benzène, le 2,5-diamino-1-(4-4-benzylpipérazinométhyl-3-thiényl)benzène, le 2,5-diamino-1-(4-(3-carboxamidophényl)aminométhyl-3-thiényl)benzène, le 2,5-diamino-1-(4-(N-méthyl-N-phényl)aminométhyl-3-thiényl)benzène, le 2,5-diamino-1-(4-diéthylaminométhyl-3-thiényl)benzène, le 2,5-diamino-1-(4-(pyridin-4-yl-méthyl)aminométhyl-3-thiényl)-benzène, le 2,5-diamino-1-(4-(3,5-diméthoxybenzyl)-aminométhyl-3-thiényl)benzène, le 2,5-diamino-1-(4-(4-morpholinophényl)aminométhyl-3-thiényl)benzène, le 2,5-diamino-1-(4-(1-(2-hydroxyéthyl))propylaminométhyl-3-thiényl)benzène, le 2,5-diamino-1-(4-cyclopropylaminométhyl-3-thiényl)benzène, le N-(2-{[4-(2,5-diaminophényl)thiophén-2-ylméthyl]amino}éthyl)acétamide, le 2,5-diamino-1-(4-cyclohexylaminométhyl-3-thiényl)-benzène, le 2,5-diamino-1-(4-propylaminométhyl-3-thiényl)benzène, le 2,5-diamino-1-(4-(o-tolyl)-aminométhyl-3-thiényl)benzène, le 2,5-diamino-1-(4-(N-méthyl-N-cyclohexyl)aminométhyl-3-thiényl)benzène, le 2,5-diamino-1-(4-(2,6-diméthylmorpholino)méthyl-3-thiényl)benzène, le 2,5-diamino-1-(4-(3,5-diméthylpipéridino)méthyl-3-thiényl)benzène, le 2,5-diamino-1(4-(3-(1-hydroxyéthyl)phényl)aminométhyl-3-thiényl)-benzène, le 2,5-diamino-1-(4-(3,4-diméthylphényl)aminométhyl-3-thiényl)benzène le 2,5-diamino-1-(4-(3-méthylmercaptophényl)aminométhyl-3-thiényl)benzène, le 2,5-diamino-1-(4-(4-diphényl)aminométhyl-3-thiényl)-benzène, le 2,5-diamino-1-(4-(4-isopropylphényl)aminométhyl-3-thiényl)benzène, le 2,5-diamino-1-(4-pentylaminométhyl-3-thiényl)benzène, le 2,5-diamino-1-(4-(dibutyl)aminométhyl-3-thiényl)benzène, le 2,5-diamino-1-(4-isopropylaminométhyl-3-thiényl)benzène, le 2,5-diamino-1-(4-(N-cyclopropylméthyl-N-propyl)aminométhyl-3-thiényl)benzène, le 2,5-diamino-1-(4-(4-hydroxy)-pipéridinométhyl-3-thiényl)benzène, le 2,5-diamino-1-(4-(fluorényl)aminométhyl-3-thiényl)benzène, le 2,5-diamino-1-(4-(4-diméthylaminophényl)aminométhyl-3-thiényl)benzène, le 2,5-diamino-1-(4-(3,5-diméthylphényl)aminométhyl-3-thiényl)benzène, le 2,5-diamino-1-(4-(4-méthoxybenzyl)aminométhyl-3-thiényl)benzène, le 2,5-diamino-1-(4-(indanyl)aminométhyl-3-thiényl)benzène, le 2,5-diamino-1-(4-(3-fluorobenzyl)aminométhyl-3-thiényl)benzène, le 2,5-diamino-1-(4-(benzyl)aminométhyl-3-thiényl)benzène, le 2,5-diamino-1-(4-(4-méthylpipérazino)aminométhyl-3-thiényl)benzène, le 2,5-diamino-1-(4-(4-tert-butylphényl)aminométhyl-3-thiényl)benzène, le 2,5-diamino-1-(4-(2-chlorobenzyl)-aminométhyl-3-thiényl)benzène, le 2,5-diamino-1-(4-(napht-1-ylméthyl)aminométhyl-3-thiényl)benzène, le 2,5-diamino-1-(4-(phényl)aminométhyl-3-thiényl)benzène, le 2,5-diamino-1-(4-(4-chlorophényl)aminométhyl-3-thiényl)benzène, le 4-{[4-(2,5-diaminophényl)thiophén-2-ylméthyl]amino}benzoate de méthyle et le 2,5-diamino-1-(3-thiényl)benzène.

7. Composition pour la teinture d'oxydation de fibres kératiniques, à base d'une combinaison de substance révélatrice-substance copulante, **caractérisée en ce qu'**elle comprend, en tant que substance révélatrice, au moins un dérivé de diaminobenzène de formule (I) selon l'une quelconque des revendications 1 à 6.

8. Composition selon la revendication 7, **caractérisée en ce que** le dérivé de diaminobenzène de formule (I) est présent en une quantité de 0,005 à 20,0 pour cent en poids.

9. Composition selon la revendication 7 ou 8, **caractérisée en ce que** la substance copulante est choisie parmi la 2,6-diaminopyridine, le 2-amino-4-[(2-hydroxyéthyl)amino]anisole, le 2,4-diamino-1-fluoro-5-méthylbenzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino]-1,5-diméthoxybenzène, la 2,3-diamino-6-méthoxypyridine, la 3-amino-6-méthoxy-2-(méthylamino)pyridine, la 2,6-diamino-3,5-diméthoxypyridine, la 3,5-diamino-2,6-diméthoxypyridine, le 1,3-diaminobenzène, le 2,4-diamino-1-(2-hydroxyéthoxy)-benzène, le 2,4-diamino-1,5-di(2-hydroxyéthoxy)benzène, le 1-(2-aminoéthoxy)-2,4-diaminobenzène, le 2-amino-1-(2-hydroxyéthoxy)-4-méthylaminobenzène, l'acide 2,4-diaminophénoxy acétique, la 3-[di(2-hydroxyéthyl)-amino]aniline, le 4-amino-2-di[(2-hydroxyéthyl)amino]-1-éthoxybenzène, le 5-méthyl-2-(1-méthyléthyl)phénol, la 3-[(2-hydroxyéthyl)amino]aniline, la 3-[(2-aminoéthyl)amino]aniline, le 1,3-di(2,4-diaminophénoxy)-propane, le di(2,4-diaminophénoxy)méthane, le 1,3-diamino-2,4-diméthoxybenzène, le 2,6-bis(2-hydroxyéthyl)aminotoluène, le 4-hydroxyindole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthylphénol, le 3-amino-2-chloro-6-méthylphénol, le 3-aminophénol, le 2-[(3-hydroxyphényl)amino]acétamide, le 5-[(2-hydroxyéthyl)amino]-2-méthylphénol, le 3-[(2-hydroxyéthyl)amino]phénol, le 3-[(2-méthoxyéthyl)-amino]phénol, le 5-amino-2-éthylphénol, le 2-(4-amino-2-hydroxyphénoxy)éthanol, le 5-[(3-hydroxypropyl)-amino]-2-méthylphénol, le 3-[(2,3-dihydroxypropyl)-amino]-2-méthylphénol, le 3-[(2-hydroxyéthyl)amino]-2-méthylphénol, la 2-amino-3-hydroxypyridine, le 5-amino-4-chloro-2-méthylphénol, le 1-naphthol, le 1,5-dihydroxynaphtalène, le 1,7-dihydroxynaphtalène, la 2,3-dihydroxynaphtaline, la 2,7-dihydroxynaphtaline, l'acétate de 2-méthyl-1-naphtalène, le 1,3-dihydroxybenzène, le 1-chloro-2,4-dihydroxybenzène, le 2-chloro-1,3-dihydroxybenzène, le 1,2-dichloro-3,5-dihydroxy-4-méthylbenzène, le 1,5-dichloro-2,4-dihydroxybenzène, le 1,3-dihydroxy-2-méthylbenzène, le 3,4-méthylènedioxyphénol, la 3,4-méthylènedioxyaniline, le 5-[(2-hydroxyéthyl)amino]-1,3-benzodioxol, le 6-bromo-1-hydroxy-3,4-méthylènedioxybenzène, l'acide 3,4-diaminobenzoïque, la 3,4-dihydro-6-hydroxy-1,4(2H)-benzoxazine, la 6-amino-3,4-dihydro-1,4(2H)-benzoxazine, le 3-méthyl-1-phényl-5-pyrazolone, le 5,6-dihydroxyindole, la 5,6-dihydroxyindoline, le 5-hydroxyindole, le 6-hydroxyindole, le 7-hydroxyindole et le 2,3-indolédinedione.

10. Composition selon l'une quelconque des revendications 7 à 9, **caractérisée en ce qu'**elle comprend, en plus du dérivé de 1,4-diaminobenzène de formule (I), en outre au moins une autre substance révélatrice qui est choisie parmi le 1,4-diaminobenzène, le 2,5-diaminotoluène, l'alcool 2,5-diaminophényléthylique, le 4-aminophénol et ses dérivés, des dérivés de 4,5-diaminopyrazole et des tétra-aminopyrimidines.

11. Composition selon l'une quelconque des revendications 7 à 10, **caractérisée en ce que** les substances révélatrices et les substances copulantes sont présentes, dans chaque cas, en une quantité totale de 0,005 à 20 pour cent en poids, par rapport à la quantité totale du colorant d'oxydation.

12. Composition selon l'une quelconque des revendications 7 à 11, **caractérisée en ce qu'**elle comprend en outre au moins un colorant direct.

13. Composition selon l'une quelconque des revendications 7 à 12, **caractérisée en ce qu'**elle présente un pH de 6,8 à 11,5.

14. Composition selon l'une quelconque des revendications 7 à 13, **caractérisée en ce qu'**elle se présente sous forme d'une solution aqueuse ou aqueuse-alcoolique, d'une crème, d'un gel ou d'une émulsion.

15. Composition selon l'une quelconque des revendications 7 à 14, **caractérisée en ce qu'**il s'agit d'un colorant capillaire.
